# EUROPÄISCHE PATENTSCHRIFT

(11) **EP 2 614 854 B1**
(45) Veröffentlichungstag und Bekanntmachung des Hinweises auf die Patenterteilung: **14.12.2016**
(21) Anmeldenummer: 13150982.0
(22) Anmeldetag: 11.01.2013
(51) Int. Cl.: A61M 37/00, A61B 17/20

(54) **Applikationsmodul und Vorrichtung zum wiederholten Aufstechen eines organischen Gewebes**
An application module and a device for the repeated piercing of an organic tissue
Module d'application et dispositif de piqûre répétée d'un tissu organique

(30) Priorität: 13.01.2012 DE 102012100308
(43) Veröffentlichungstag der Anmeldung: 17.07.2013
(73) Patentinhaber: Technische Universität Berlin, 10623 Berlin (DE)
(72) Erfinder: Scherkowski, Dirk, 12437 Berlin (DE)
(74) Vertreter: Bittner, Thomas L.

(56) Entgegenhaltungen:
- WO-A1-2008/010573
- CN-U- 201 658 748
- US-A- 3 010 455
- US-A1- 2011 245 776
- US-B1- 6 743 211

## Beschreibung

Die Erfindung betrifft ein Applikationsmodul für eine Vorrichtung zum wiederholten Aufstechen eines organischen Gewebes und eine Vorrichtung zum wiederholten Aufstechen eines organischen Gewebes.

### Hintergrund

Organische Gewebe können in verschiedenen Formen auftreten. So bildet insbesondere die menschliche oder tierische Haut ein organisches Gewebe, das eine wirkungsvolle Barriere gegen das Eindringen von höhermolekularen Stoffen oder von Mikroorganismen in den menschlichen oder tierischen Körper darstellt. Um dennoch therapeutische Wirkstoffe oder andere Stoffe, kosmetische Substanzen oder - wie beim Tätowieren oder beim Permanent-Make-up - Farbe, Farbstoffe oder Pigmente über die Haut im Körper einbringen zu können, ist es bekannt, die Haut, insbesondere die äußere Schicht der Haut, die Epidermis, durch mithilfe geeigneter auf die Haut wirkender Schneid- oder Stechwerkzeuge (Penetrierwerkzeuge) erzeugte Löcher oder Schlitze (Aufstechen) für die jeweiligen Wirkstoffe oder Pigmente durchlässig zu machen (vgl. zum Beispiel EP 2 011 539 B1, US 2009/0125 050 A1).

Aus dem Dokument JP 2000 342 332 A ist eine Tätowiervorrichtung bekannt, bei der zur Hautpenetration ein Nadelbündel aus endseitig miteinander verlöteten Nadeln vorgesehen ist. Im Betrieb werden die Nadeln als Bündel gemeinsam vor und zurück bewegt. Stechspitzen der Nadeln im Bündel können voneinander weg gebogen sein.

Das Dokument US 2005 / 0209566 A1 offenbart eine Vorrichtung zum Applizieren eines Wirkstoffes auf ein biologisches Gewebe. Das Stechwerkzeug führt im Betrieb eine lineare Vor- und Rückwärtsbewegung aus, die von einer Schwenkbewegung überlagert wird.

Aus dem Dokument US 2011/0245776 A1 ist eine Vorrichtung zum Aufstechen eines organischen Gewebes bekannt. In einer Ausführungsform ist die Vorrichtung ausgebildet, in eine Körperöffnung eingeführt zu werden, beispielsweise die Luftröhre, um hierin dann auf gegenüberliegenden Seiten der Luftröhre Applikatoren mit Mikronadeln anzuwenden, so dass in das Gewebe der Luftröhre eingestochen wird. Die Applikatoren mit den Mikronadeln sind an Schwenkarmen aufgenommen, die ihrerseits an einem Grundkörpern gelagert sind, mit dem die Applikatoren in den Körper eingeführt werden.

Im Dokument US 6,743,211 B1 ist eine Mikronadel-Vorrichtung zum Aufstechen einer Haut offenbart. Die Mikronadeln sind entweder parallel zueinander ausgerichtet oder verlaufen mit ihren distalen Enden sternförmig auseinander.

Eine Vorrichtung zum lokalen Aufstechen einer Haut ist weiterhin aus dem Dokument US 3,010,455 bekannt. Hierbei ist an der Vorrichtung eine einzelne Stechnadel vorgesehen.

Vorrichtungen zum Aufstechen einer Haut sind weiterhin in den Dokumenten CN 201658748 U sowie WO 2008/010573 offenbart.

Ein Aufstechen und / oder Aufschneiden von Haut wird auch ohne begleitendes Applizieren eines Wirkstoffes zu medizinischen oder kosmetischen Zwecken genutzt werden. Hierbei werden üblicherweise Schneid- oder Stechwerkzeuge genutzt, die zum wiederholten Penetrieren des Gewebes an einen maschinellen Antrieb koppeln, welcher die Penetrierwerkzeuge mit einer Betriebsfrequenz antreibt.

### Zusammenfassung

Aufgabe der Erfindung ist es, ein Applikationsmodul für eine Vorrichtung zum wiederholten Aufstechen eines organischen Gewebes und eine Vorrichtung anzugeben, die eine einfache Handhabung bei der Nutzung unterstützen und ein schonendes und möglichst schmerzarmes Penetrieren des organischen Gewebes, insbesondere einer tierischen oder menschlichen Haut, ermöglichen.

Diese Aufgabe wird erfindungsgemäß durch ein Applikationsmodul für eine Vorrichtung zum wiederholten Aufstechen eines organischen Gewebes nach dem unabhängigen Anspruch 1 gelöst. Weiterhin ist eine Vorrichtung zum wiederholten Aufstechen eines organischen Gewebes nach dem Anspruch 13 geschaffen. Vorteilhafte Ausgestaltungen sind Gegenstand von abhängigen Unteransprüchen.

Die vorgeschlagenen Technologien ermöglichen ein flexibles und schonendes Penetrieren eines organischen Gewebes, insbesondere einer tierischen oder menschlichen Haut, wobei das Penetrieren das Eindringen der Penetriereinrichtung in das organische Gewebe betrifft.

Die Anordnung der Applikationswerkzeuge bei der Vorrichtung, die jeweils an dem Betätigungsmechanismus aufgenommen sind, ist zwischen einer Ausgangsstellung und einer Penetrierstellung verlagerbar. Ein oder mehrere der Applikationswerkzeuge sind am distalen Werkzeugende mit einer jeweiligen Penetriereinrichtung versehen. Der Übergang zwischen der Ausgangsstellung und der Penetrierstellung, also der Stellung, in welcher die Anordnung von Applikationswerkzeugen dem Zustand entspricht, in dem im Betrieb das organische Gewebe penetriert ist, erfolgt im einfachsten Fall dadurch, dass lediglich eines der Applikationswerkzeuge in seiner Relativstellung zu den verbleibenden Applikationswerkzeugen verlagert wird.

Hierzu ist es möglich, dass entweder das eine Applikationswerkzeug oder die verbleibenden Applikationswerkzeuge mit Hilfe des Betätigungsmechanismus verlagert werden. Es erfolgt hierdurch ein Übergang aus der Ausgangsstellung der Anordnung von Applikationswerkzeugen in die Penetrierstellung der Anordnung. Gleichzeitig kann den einzelnen Applikationswerkzeugen insoweit jeweils eine Ausgangsstellung und eine Penetrierstellung zugeordnet werden, insbesondere in Relation zu dem einen oder den mehreren verlagerten Applikationswerkzeugen. Die Penetrierstellung bedeutet im Betrieb, dass das distale Applikationsende wenigstens eines der Applikationswerkzeuge der Anordnung, welches mit einer Penetriereinrichtung versehen ist, in das organische Gewebe eingedrungen ist.

Weiterhin ist eine maschinelle Antriebseinrichtung vorgesehen, die an den Betätigungsmechanismus koppelt und geeignet ist, das relative Verlagern des wenigstens einen Applikationswerkzeuges zwischen der Ausgangsstellung und der Penetrierstellung wiederholt mit einer Betriebsfrequenz zu bewirken. Die Betriebsfrequenz der von der Antriebseinrichtung bereitgestellten wiederholten (repetierenden) Antriebsbewegung kann etwa 5Hz bis etwa 200Hz betragen.

Die Vorrichtung kann modular aufgebaut sein. Zum Beispiel kann die Penetriereinrichtung in einem Applikationsmodul (Stechmodul) angeordnet sein, welches seinerseits lösbar an ein Antriebsmodul koppelt, welches die Antriebseinrichtung aufnimmt. An dem Antriebsmodul kann an Handgriff gebildet sein, welcher vom Benutzer im Betrieb mit den Fingern oder der Hand gegriffen wird. Auch kann das Antriebsmodul ein Handstück bilden. Wenn eine Spendeneinrichtung vorgesehen ist, so kann diese am Applikationsmodul oder an dem Antriebsmodul angeordnet sein.

Das Applikationsmodul verfügt über eine Kopplungseinrichtung, vorzugsweise im Bereich eines rückseitigen Modulendes, mittels der es an eine Antriebseinrichtung koppelbar ist, insbesondere derart, dass die von der Antriebseinrichtung bereitgestellte Antriebskraft in einen Betätigungsstößel eingekoppelt wird, wenn Applikationsmodul und Antriebseinrichtung gekoppelt sind, zum Beispiel über eine Schraub- und / oder eine Steckverbindung.

Bei der Verwendung der Vorrichtung wird diese üblicherweise auf das organische Gewebe in einem Bereich aufgesetzt, in welchem mittels Penetrieren ein oder mehrere Gewebeöffhungen erzeugt werden sollen. Beim Nutzen der Vorrichtung entsteht beim Penetrieren in dem organischen Gewebe eine Öffnung, welche zum Beispiel zum Einbringen eines oder mehrerer Wirkstoffe in das organische Gewebe genutzt werden kann. In einer anderen Ausführung dient das Penetrieren des Gewebes zum Beispiel einer Hautstimulierung.

Beim Verwenden der Vorrichtung zur Wirkstoffapplikation (Applikationsvorrichtung) kann das Ausbringen eines oder mehrerer Wirkstoffe aus einer Spendeeinrichtung auf das organische Gewebe vor, nach und / oder während des Penetrierens vorgesehen sein, wobei das Einbringen in das organische Gewebe während des und / oder nach dem Penetrieren(s) geschieht.

Zu den applizierbaren Wirkstoffen, welche über eine Gewebeöffnung, zum Beispiel eine Hautöffnung, in das Gewebe eingebracht werden können, gehören Farbe, Farbstoffe oder Pigmente in Verbindung mit dem Ausbilden eines Tattoos oder von permanentem Make-up, kosmetische Wirkstoffe und medizinische Wirkstoffe, beispielsweise zum Impfen. Zu den fluiden Substanzen, die unter Nutzung der Vorrichtung appliziert werden können, gehören beispielsweise Lösungen, Emulsionen, kolloidale Lösungen, Dispersionen, Suspensionen, Lotionen, Cremes, Gele, feste oder partikuläre Substanzen wie beispielsweise Pigmente, Partikel, Mikropartikel, Nanopartikel, Mikrokapseln oder biologische Substanzen wie beispielsweise Proteine, Peptide, Enzyme, Nucleinsäuren, Gene, Vektoren, Zellen, Haarwurzeln, (abgetötete) Viren, Bakterien aber auch Substanzen wie Vesikel, Mizellen oder Nanoroboter.

Die Vorrichtung kann für die verschiedenen vorgenannten Anwendungen verwendet werden.

Die Vorrichtung zum wiederholten Aufstechen eines organischen Gewebes kann in einer Ausgestaltung der Erfindung als ein Handgerät ausgeführt sein.

Die Vorrichtung oder einzelne Modulelemente hiervon, zum Beispiel das an die Antriebseinrichtung koppelnde Applikations- oder Stechmodul, können als Einwegartikel ausgeführt sein.

Die Spendeeinrichtung, die konfiguriert ist, einen zu applizierenden Wirkstoff bereitzustellen und bei der Anwendung der Vorrichtung zur Gewebepenetration dann auszubringen, kann beispielsweise mit einem Vorratsbehälter gebildet sein, welcher in Fluidverbindung mit einer Ausbringöffnung steht, über die der Wirkstoff ausgebracht werden kann. Derartige Spendeeinrichtungen sind als solche in verschiedenen Ausführungen bekannt, zum Beispiel in Verbindung mit Geräten zum Ausbilden von Tattoos oder permanentem Make-up. Hierbei kann vorgesehen sein, dass das Betätigen der Spendeeinrichtung zum Ausbringen oder Ausgeben des Wirkstoffes gleichzeitig mit dem Betätigen der Applikationswerkzeuge erfolgt, beispielsweise dadurch, dass die Applikationswerkzeuge auf die Spendeeinrichtung einwirken, zum Beispiel mittels einer Druckbeaufschlagung, vorzugsweise auf einen Vorratsbehälter mit dem Wirkstoff. Alternativ oder ergänzend kann vorgesehen sein, dass die Spendeeinrichtung zum Ausbringen des Wirkstoffes unabhängig und getrennt von den Applikationswerkzeugen zu betätigen ist.

Die Vorrichtung vermeidet, dass das Gewebe aufgrund seiner Elastizität und Flexibilität beim Ansetzen der Applikationswerkzeuge auf der Gewebeoberfläche ausweicht, indem das Gewebe durch die distalen Werkzeugenden der Applikationswerkzeuge vorgespannt wird. Es wird eine hohe sowie auch insbesondere eine niedrige Eindringgeschwindigkeit der Applikationswerkzeuge unterstützt. Insgesamt wird ein schonendes und schmerzarmes Penetrieren von organischem Gewebe gefördert.

Der konstruktive Aufbau der Vorrichtung ist derart, dass beim Übergang zwischen der Ausgangsstellung und der Penetrierstellung der Anordnung von Applikationswerkzeugen wenigstens ein Applikationswerkzeug, welches relativ zu einem oder mehreren verbleibenden Applikationswerkzeugen der Anordnung verlagert wird, hinsichtlich seiner aufgegebenen Applikationskraft gegenläufig zur Wirkrichtung einer von einem anderen Applikationswerkzeug der Anordnung bereitgestellten Applikationskraft wirkt. Bei einer Kraftkomponentenzerlegung sind so zwei parallele, aber entgegengesetzt gerichtete Kraftkomponenten gegeben, nämlich eine Kraftkomponente der Applikationskraft des wenigstens einen Applikationswerkes und eine entgegengesetzte Kraftkomponente der Applikationskraft des anderen Applikationswerkes.

Die Gegenläufigkeit der Wirkrichtungen der bereitgestellten Applikationskräfte sowie der Umstand, dass sich in einer möglichen Ausgestaltung das wenigstens eine und das andere Applikationswerkzeug zumindest in der Penetrierstellung kreuzen, bedeutet im Betrieb, dass sich die Applikationswerkzeuge bei einem möglichen Ausführungsbeispiel dieser Ausgestaltung, zumindest deren distale Werkzeugenden, beim Übergang in die Penetrierstellung voneinander entfernen, was zu einem Spannen der Gewebeoberfläche vor und wahlweise auch noch während des Penetrierens führt. In einem anderen Ausführungsbeispiel kreuzen sich rückseitige Verlängerungslinien der distalen Werkzeugenden der Applikationswerkzeuge.

Insbesondere diese Ausführungen, bei denen das Verlagern der Anordnung von Applikationswerkzeugen aus der Ausgangsstellung in die Penetrierstellung im Betrieb ein Spannen der Oberfläche des organischen Gewebes bewirkt, unterstützt ein möglichst schonendes Penetrieren des Gewebes. Im Fall des Penetrierens einer menschlichen Haut wird diese Art der Führung der Applikationswerkzeuge als relativ schmerzarm empfunden. Dieses schmerzarme Penetrieren der Haut kann sich auch als vorteilhaft erweisen, beispielsweise beim Penetrieren einer menschlichen oder einer tierischen Haut, beim Tätowieren dieser oder beim Verabreichen von Medikamenten.

Den Applikationswerkzeugen kann bei der Nutzung der Vorrichtung zum Penetrieren des organischen Gewebes nach dem Aufsetzen der distalen Werkzeugenden der Applikationswerkzeuge auf das organische Gewebe und dem Betätigen des Betätigungsmechanismus zur Relativverlagerung aus der Ausgangsstellung in die Penetrierstellung ein jeweiliger Wirkbereich auf der Oberfläche des organischen Gewebes zugeordnet werden. Der Wirkbereich eines Applikationswerkzeuges erstreckt sich über den Oberflächenabschnitt des organischen Gewebes, in welchem die vom jeweiligen Applikationswerkzeug auf die Gewebeoberfläche eingeleitete Applikationskraft sich auswirkt, insbesondere in Form einer oberflächigen Gewebeverschiebung. Die Wirkbereiche für die Applikationswerkzeuge der Anordnung bei der Vorrichtung überlappen sich zumindest für die einander zugeordneten Applikationswerkzeuge, so dass die gegenläufige Wirkrichtung der Applikationskräfte für die einander zugeordneten Applikationswerkzeuge gegenläufige Effekte in den sich überlappenden Wirkbereichen hervorruft, zum Beispiel gegenläufige Kräfte zur Hautoberflächenverschiebung. Bevorzugt gleichen sich diese Applikationskräfte für benachbarte Applikationswerkzeuge, die gegenläufig arbeiten, auf dem organischen Gewebe im Wesentlichen aus, so dass die oberflächige Verschiebung von Schichten des organischen Gewebes, beispielsweise der Haut, vermieden oder zumindest weitestgehend gemindert ist. Bevorzugt gleichen sich die Applikationskräfte zweier gegenläufig arbeitender Applikationswerkzeuge, die benachbart zueinander auf dem Gewebe aufsetzen, auf dem organischen Gewebe im Wesentlichen aus, so dass die oberflächige Verschiebung von Schichten des organischen Gewebes, beispielsweise der Haut, vermieden oder zumindest weitestgehend gemindert ist.

Die Applikationswerkzeuge koppeln an den Betätigungsmechanismus, derart, dass sie funktionsgerecht mittels Betätigung des Mechanismus eingesetzt werden können, sei es per Hand oder mittels eines maschinellen Antriebs. Der maschinelle Antriebsmechanismus, der zum Beispiel unter Verwendung eines elektrischen Motors gebildet ist, kann selbst lösbar am Betätigungsmechanismus montiert sein.

Der Betätigungsmechanismus kann in einer Ausführungsform auch selbstauslösend sein, beispielweise dadurch, dass ein vorgespanntes Werkzeug aus der vorgespannten Stellung gelöst wird.

Der Betätigungsmechanismus kann konfiguriert sein, mehrere oder alle Applikationswerkzeuge beim Übergang zwischen der Ausgangsstellung und der Penetrierstellung gemeinsam zu verlagern. Auch ein Verlagern von mehreren Gruppen von Applikationswerkzeugen kann vorgesehen sein.

Auch können in einer Ausgestaltung mehrere oder alle distalen Werkzeugenden der Applikationswerkzeuge mit einer Penetriereinrichtung versehen sein. Die Penetriereinrichtung ist konfiguriert, das organische Gewebe mittels einer Schnitt- und / oder einer Stechbewegung zu öffnen. Bei der Ausgestaltung, bei der nicht alle Applikationswerkzeuge mit einer Penetriereinrichtung versehen sind, können einzelne oder alle der verbleibenden Applikationswerkzeuge im distalen Endbereich stumpf ausgebildet sein.

Die distalen Werkzeugenden der Applikationswerkzeuge können in der Anordnung von Applikationswerkzeugen entlang einer Anordnungslinie angeordnet sein, bei der es sich um eine gerade Linie oder auch um eine krumme Linie handelt, insbesondere eine Ringlinie.

Eine Weiterbildung sieht vor, dass das wenigstens eine und das andere Applikationswerkzeug sich bei der Verlagerung zu der Penetrierstellung hin kreuzen. Bei dieser Ausgestaltung kommt es bereits auf dem Verlagerungsweg zu der Penetrierstellung hin, vorzugsweise auf der zweiten Hälfte des Verlagerungsweges, zum Kreuzen des wenigstens einen Applikationswerkzeuges, welches relativ zum verbleibenden verlagert wird, und dem anderen Applikationswerkzeug aus der Anordnung. Die sich kreuzende Anordnung der Applikationswerkzeuge bleibt dann auch in der Penetrierstellung erhalten. Es kann auch vorgesehen sein, dass sich das wenigstens eine Applikationswerkzeug und das andere Applikationswerkzeug bereits in der Ausgangsstellung kreuzen. Bei dieser Ausgestaltung werden die distalen Werkzeugenden dann bei der Verlagerung in die Penetrierstellung voneinander weg bewegt, wenigstens die Spitzen der Werkzeugenden, so dass die distalen Werkzeugenden auseinander laufen und im Betrieb insoweit das organische Gewebe im Applikationsbereich spannen oder auseinander ziehen. Hinsichtlich der kreuzenden Anordnung des wenigstens einen und des anderen Applikationswerkzeuges kann vorgesehen sein, dass die sich kreuzende Anordnung in einer Ausgestaltung erst dann gebildet wird, wenn die Applikationswerkzeuge beim Verlagern in die Penetrierstellung ein Gerätegehäuse durch eine Gehäuseöffnung verlassen. Ein solches Gehäuse kann insbesondere bei einer Vorrichtung vorgesehen sein, welche einen maschinellen Antrieb aufweist. Die Applikationswerkzeuge oder rückwärtigen Verlängerungslinien der distalen Werkzeugenden sind also innerhalb des Gerätegehäuses nicht gekreuzt, sondern nur außerhalb.

Bei einer Ausgestaltung kann vorgesehen sein, dass das wenigstens eine und das zugeordnete Applikationswerkzeug bei der Verlagerung zu der Penetrierstellung hin seitlich versetzt zueinander geführt sind. Bezogen auf eine Anordnungslinie der distalen Werkzeugenden der Applikationswerkzeuge, insbesondere der distalen Spitzen, besteht bei dieser Ausgestaltung ein seitlicher Versatz zueinander bereits bei der Verlagerungsbewegung zur Penetrierstellung hin, also nicht nur in der Penetrierstellung selbst. Auch kann vorgesehen sein, dass der seitliche Versatz der einander zugeordneten Applikationswerkzeuge bereits in der Ausgangsstellung vorhanden ist.

Eine Ausführungsform sieht vor, dass mittels des Betätigungsmechanismus die Verlagerung zwischen der Ausgangsstellung und der Penetrierstellung repetierend ausführbar ist. Die repetierende Ausführbarkeit der Verlagerung bedeutet, dass die Anordnung von Applikationswerkzeugen mehrfach und wiederholt zwischen den Stellungen verlagerbar ist. Abweichend hiervon würde sich beispielsweise eine Einmalklammer verhalten, die einer einmaligen Befestigung dient. Die repetierende Verlagerung wird insbesondere bei einer Vorrichtung ausgeführt, welches über einen maschinellen Antrieb verfügt, wie es zum Beispiel bei Handgeräten zum Tätowieren oder zum Ausbilden von permanentem Make-up der Fall ist. Des Weiteren wird die repetierende Verlagerung bei einer Vorrichtung beim Herausführen beispielsweise einer Hohlnadel, die zum Applizieren eines Impfstoffes dienen kann, ausgeführt.

Es kann vorgesehen sein, dass eine Bewegungsbahn bei der Verlagerung des wenigstens einen Applikationswerkzeuges relativ zu dem oder den verbleibenden Applikationswerkzeugen der Anordnung eine Kreisbahnbewegung und / oder eine geradlinige Bewegung umfasst. Das Ausführen einer Kreisbahn- oder Kreisbahnabschnittsbewegung kann mittels einer Rotations- oder einer Schwenkbewegung ausgeführt werden, wofür das wenigstens eine Applikationswerkzeug dann drehbar oder schwenkbar an den Betätigungsmechanismus koppelt. Eine geradlinige Bewegung kann insbesondere in Verbindung mit einer geraden (nicht gekrümmten) Nadel oder einer solchen Nadelgruppen für die Verlagerung zwischen der Ausgangsstellung und der Penetrierstellung vorgesehen sein. Beispielsweise können Nadeln in einer zugeordneten Nadelführung gelagert sein.

Bei einer Ausgestaltung kann vorgesehen sein, dass die Penetriereinrichtung des wenigstens einen Applikationswerkzeuges zumindest in einem letzten Abschnitt der Verlagerungsbewegung zur Penetrierstellung hin entlang einer Bewegungsrichtung geführt wird, die einen vom rechten Winkel verschiedenen Winkel mit einer den distalen Werkzeugenden der Applikationswerkzeuge im Betrieb zugeordneten Auflagefläche einnimmt. Die Auflagefläche wird bei der Nutzung des Handgerätes von der Oberfläche des organischen Gewebes gebildet. Dass Heranführen der Penetriereinrichtung am distalen Werkzeugende in einem spitzen Winkel auf das organische Gewebe zu unterstützt ein möglichst sanftes Eindringen der Penetriereinrichtung in die Gewebeoberfläche. Im Fall einer krummlinigen Bewegung der Penetriereinrichtung auf die Gewebeoberfläche zu bezieht sich der spitze Winkel auf den Winkel zwischen der Gewebeoberfläche und der Tangente an die Bewegungsbahn.

Eine Weiterbildung kann vorsehen, dass das wenigstens eine Applikationswerkzeug bei der Verlagerungsbewegung zwischen der Ausgangsstellung und der Penetrierstellung wenigstens abschnittsweise in einer zugeordneten Werkzeugführung geführt wird. Beispielsweise kann vorgesehen sein, Einzelnadeln oder Nadelgruppen in einer Nadelführung aufzunehmen und bei der Verlagerung zu führen, zum Beispiel in rillenartigen oder kanalförmigen Ausnehmungen. Die Führung kann auf dem gesamten Weg zwischen der Ausgangsstellung und der Penetrierstellung oder auch nur auf einem oder mehreren Teilabschnitten dieses Weges gegeben sein.

Eine Weiterbildung sieht vor, dass das wenigstens eine Applikationswerkzeug oder das zugeordnete Applikationsgegenwerkzeug mit einem Kammbauteil gebildet ist, an dem mehrere nebeneinander liegende Applikationselemente angeordnet sind. In einer Ausführungsform verfügen die mehreren nebeneinander liegenden Applikationselemente jeweils über eine Penetriereinrichtung, zum Beispiel verfügen die Kammelemente also über eine Stecheinrichtung. In einer Ausgestaltung greifen zwei einander gegenüberliegend angeordnete Kammbauteile wenigstens in der Penetrierstellung kreuzend ineinander. Dieses führt dazu, dass die Oberfläche des zu penetrierenden Gewebes über die gesamte Kammbreite gespannt oder gestreckt wird, wenn die Kammbauteile bei der Nutzung des Handgeräts in die Penetrierstellung verlagert werden. Bei einer Ausgestaltung verfügt die Vorrichtung über zwei Applikationswerkzeuge, die jeweils als Kammbauteil ausgeführt sind, bei denen sämtliche distalen Werkzeugenden jeweils eine Penetriereinrichtung aufweisen. Eine weitere Ausführungsform sieht eine Vorrichtung mit zwei Applikationswerkzeugen vor, die jeweils als ein mehrreihiges Kammbauteil gebildet sind, wobei auch hier vorgesehen sein kann, dass sämtliche distalen Werkzeugenden jeweils eine Penetriereinrichtung aufweisen, zum Beispiel in Form einer Nadelspitze. Auf dem Gebiet des Tätowierens werden mehrreihige Nadelanordnungen auch als "Magnum"-Anordnung bezeichnet.

Eine Ausführungsform sieht vor, dass ein Hub der Verlagerungsbewegung des wenigstens einen Applikationswerkzeuges einstellbar ist. Das Einstellen des Hubes der Verlagerungsbewegung kann genutzt werden, um eine Penetriertiefe am zu penetrierenden organischen Gewebe anwendungsbezogen einzustellen.

Eine Fortbildung kann vorsehen, dass im Bereich der Anordnung von Applikationswerkzeugen und / oder benachbart hierzu eine Gewebeanschlag- oder Gewebeführungsfläche gebildet ist. Im Fall des Anordnens der Applikationswerkzeuge in einem Gerätegehäuse können derartige Gewebeanschlag- oder Gewebeführungsflächen beispielsweise benachbart zu einer Gehäuseöffnung gebildet sein, durch welche hindurch die Applikationswerkzeuge beim Übergang in die Penetrierstellung das Gehäuse verlassen. Die Gewebeanschlag- oder Gewebeführungsflächen dienen dann insbesondere der verbesserten Führung des Handgerätes über die Oberfläche des organischen Gewebes, welches zu penetrieren ist. Auch kann vorgesehen sein, dass die Gewebeanschlag- oder Gewebeführungsflächen mit Ausnehmungen oder Rücksprüngen gebildet sind, zum Beispiel zur Aufnahme von Gewebefalten, die sich bei der Nutzung des Handgerätes zum Penetrieren des organischen Gewebes dort gegebenenfalls vorübergehend im Oberflächenbereich bilden. In einer Ausgestaltung kann vorgesehen sein, dass die Gewebeanschlag- oder Gewebeführungsfläche einstellbar ist, beispielsweise mittels einer Relativverlagerung in Relation zu dem Gerätegehäuse. So kann vorgesehen sein, dass derartige Flächen aus- und eingefahren werden können. In einer bevorzugten Ausführungsform ist die Gewebeeindringsperre gleichzeitig auch mit einer Werkzeugführung ausgebildet.

Bei einer Ausgestaltung kann vorgesehen sein, dass die Applikationswerkzeuge der Anordnung an einem gemeinsamen Basisbauteil gebildet sind. Bei dieser oder anderen Ausführungen kann vorgesehen sein, die Applikationswerkzeuge mit einer Ringnadel, einer Ringhohlnadel oder unter Verwendung eines Ringflachmaterials auszubilden. Unabhängig hiervon kann beispielsweise vorgesehen sein, zwei Applikationswerkzeuge an den Enden eines Drahtbügels herzustellen, der in einem Gehäuse aufgenommen ist, derart, dass mittels Zusammendrücken des Drahtbügels, sei es per Hand oder mittels maschinellen Antriebs, die an den Enden gebildeten Applikationswerkzeuge aus der Ausgangsstellung in die Penetrierstellung verlagert werden. So kann für den Handbetrieb eine Vorrichtung mit einem Flachgehäuse gebildet sein, auf dessen Seiten Bügelabschnitte überstehen, die der Nutzer per Hand, also mittels zweier Finger, zusammendrücken kann, worauf auf der Gehäuseunterseite die beiden Applikationswerkzeuge zum Penetrieren des organischen Gewebes ausgefahren werden, derart, dass sie sich zumindest in der Penetrierstellung kreuzen.

Eine Weiterbildung sieht vor, dass das distale Werkzeugende der Applikationswerkzeuge zumindest in der Ausgangsstellung ganz oder teilweise in ein Gerätegehäuse eingefahren ist. Eine solche Ausgestaltung kann sowohl bei einer Vorrichtung mit einem maschinellen Antrieb als auch bei einer Vorrichtung vorgesehen sein, bei dem der Betätigungsmechanismus per Hand zu bedienen ist, also manuell. Ein möglicher störender Einfluss der distalen Werkzeugenden der Applikationswerkzeuge, wenn diese sich in der Ausgangsstellung außerhalb des Gehäuses befinden, wird so gemindert. Beispielsweise unterstützt ein vollständiges Einfahren der Applikationswerkzeuge in das Gerätegehäuse ein leichtes Verschieben des Handgerätes über die Oberfläche des organischen Gewebes. Ein Festhängen aufgrund des Verhakens der distalen Werkzeugenden am Gewebe ist verhindert. Auch wird die Verletzungsgefahr während der Nichtnutzung des Handgerätes gemindert.

Bei einer Ausgestaltung kann vorgesehen sein, dass an den Betätigungsmechanismus eine maschinelle Antriebseinrichtung koppelt, die konfiguriert ist, die Verlagerungsbewegung des wenigstens einen Applikationswerkzeuges zwischen der Ausgangsstellung und der Penetrierstellung wiederholt mit einer Betriebsfrequenz zu bewirken. Vorzugsweise ist die Betriebsfrequenz einstellbar. Bei der Vorrichtung mit der maschinellen Antriebseinrichtung ist es ermöglicht, das organische Gewebe, zum Beispiel eine Haut, mehrfach hintereinander aufzustechen oder aufzuschneiden, insbesondere auch für eine flächige Anwendung. Vor, während und / oder nach dem Penetrieren des organischen Gewebes kann dann auf den Applikationsbereich, in dem die Gewebeöffnungen hergestellt werden, ein Wirkstoff oder eine Substanz aufgebracht werden, zum Beispiel eine Farbe, ein Farbstoff oder Pigment, ein kosmetischer und / oder ein medizinischer Wirkstoff, welcher über die mit Hilfe der Vorrichtung erzeugten Gewebeöffhungen dann eindringen kann, zum Beispiel in eine menschliche oder tierische Haut. Die Einstellbarkeit der Betriebsfrequenz unterstützt hierbei ein optimiertes Applizieren für unterschiedliche Wirkstoffe.

Im Zusammenhang mit dem Verfahren zum wiederholten Aufstechen eines organischen Gewebes gelten die vorangehend gemachten Erläuterungen entsprechend. Das Verfahren kann als Verfahren zum Ausbringen eines Wirkstoffes mittels der Vorrichtung zum Applizieren eines Wirkstoffes auf oder in ein organisches Gewebe ausgeführt werden.

Der Begriff organisches Gewebe in der hier verwendeten Bedeutung umfasst insbesondere Gewebeausbildungen aus der folgenden Gruppe: Gewebeschichten, Haut (Epidermis und / oder Dermis), Schleimhaut, Endothel, Gefäßwände und deren Schichten (Intima, Media, Adventitia), Hirnhaut, Herzbeutel, Knochenhaut, Peritoneum und andere seröse Haut, Gelenkkapsel, innere und / oder äußere Lumina oder Oberflächen begrenzende Gewebeschichten, unabhängig davon, ob diese über einen natürlichen oder künstlichen Zugang erreichbar sind, tierisches Gewebe, humanes Gewebe, biologisches Gewebe und implantierbare Materialien.

Das Penetrieren des organischen Gewebes zum Applizieren einer oder mehrerer Substanzen kann in vielen Anwendungsbereichen eingesetzt werden. Beispielsweise im Bereich Körperkunst, insbesondere das Einbringen von Farbe, Pigmenten oder Farbstoffen in die Haut beim Tätowieren oder beim permanenten Make-up. Ebenso kann das Applizieren von kosmetischen Substanzen durchgeführt werden, beispielsweise Bleichmittel oder Substanzen gegen Hautalterung (Anti-Aging Mittel) oder zur Faltenminderung, wie beispielsweise Botox oder Haut straffende Lotionen. Des Weiteren kann die Applikation von medizinischen Substanzen auf oder in ein organisches Gewebe, insbesondere in die Haut, beispielsweise zur epidermalen Insulintherapie oder Mesotherapie vorgesehen sein.

Die manuelle oder maschinelle Einmalpenetration von Gewebe, insbesondere von Haut, kann beispielsweise bei einem epi- oder intradermalen Impf-, Immunisierungs-, Hyposensibilisierungs- oder diagnostischem Testverfahren, zum Beispiel beim so genannten PRICK-Test auf Allergien, vom Geschick des Anwenders unabhängig schmerzfrei, sicher unblutig, weniger die Haut reizend und damit die Aussagekraft oder Verlässlichkeit des Verfahrens steigernd eingesetzt werden.

Mit Hilfe der Vorrichtung lassen sich in einer Verwendung Haarwurzeln implantieren. Hierbei werden in den mittels der Penetriereinrichtung erzeugten Gewebeöffhungen die Haarwurzeln aus der Spendeneinrichtung eingebracht.

Die Applikationswerkzeuge können auf einer Seite der Anordnungslinie auch stumpf ausgebildet sein und nur als Halte- oder Spannelement auf dieser einen Seite auf das Gewebe wirken, während nur die von der gegenüberliegenden Seite in im Wesentlichen entgegengesetzter Richtung wirkenden Werkzeuge scharf ausgebildet sind und in das Gewebe eindringen.

Ein von gegeneinander wirkenden Applikationswerkzeugen eingeschlossener Winkel α kann in der Projektionsebene quer zur Wirkrichtung der Applikationswerkzeuge kleiner als 180° und größer als 0° sein und in einer bevorzugten Ausgestaltung zwischen 130° und 150° liegen. Ein spitzer Winkel β zwischen auf das organische Gewebe wirkenden Applikationswerkzeugen und der Gewebeoberfläche kann auf beiden Seiten gleich groß oder unterschiedlich groß sein.

Eine Mehrzahl von beidseitig entlang der oben erwähnten Anordnungslinie nebeneinander ausgerichteten und in entgegengesetzter Kraftrichtung wirkenden Applikationswerkzeugen können jeweils einen Werkzeugkamm bilden, wobei die Anordnungslinie oder der Verlauf der Spitzen des Werkzeugkamms gerade oder durch winklig zueinander angeordnete Geraden definiert und andererseits auch ein- oder mehrfach gekrümmt sein kann. Die Werkzeugspitzen der Applikationswerkzeuge sind unmittelbar vor dem Penetrieren beim Gewebekontakt bevorzugt entweder vor oder hinter der Anordnungslinie oder unmittelbar auf der Anordnungslinie positioniert. In weiterer Ausgestaltung können die an einem Werkzeugträger angebrachten Werkzeugkämme aus einzelnen Applikationswerkzeugen gefertigt oder jeweils einstückig ausgebildet sein.

Die Penetriereinrichtungen können beispielsweise mittels Nadeln oder Mikronadeln ausgeführt sein, wobei auch Hohlnadeln zum Einsatz kommen können. Auch Schneidklingen quer oder längs der Kraftwirkungsrichtung des jeweils zugeordneten Applikationswerkzeuges verlaufend zum Schneiden des Gewebes können zum Einsatz kommen. Weiterhin können Bifurkationsnadeln verwendet werden.

Eine manuell betriebene Applikationsvorrichtung kann zwei etwa V-förmig angeordnete und federnd miteinander verbundene, mit den Fingern des Benutzers betätigte Betätigungsarme mit an den freien Enden angeformten Applikationswerkzeugträgern und an diesen befestigten Applikationswerkzeugen aufweisen. Eine derart manuell betätigte Vorrichtung zum Penetrieren kann mit allen Funktionselementen (Betätigungsarme, Werkzeugträger, Penetriereinrichtungen und / oder Werkzeugkämme) aber auch aus einem Stück, wie zum Beispiel einem etwa V-förmig, ringförmig oder rechteckig gestalteten elastischen Flachmaterial, gefertigt sein.

Die manuell oder maschinell angetriebenen Betätigungsarme können mit an den freien Enden vorgesehenen Applikationswerkzeugen in einem Gehäuse angeordnet sein. Die Applikationswerkzeuge befinden sich zum Beispiel im Bereich einer Öffnung des Gehäuses und sind an die Öffnung begrenzenden äußeren Werkzeugführungselementen sowie auf einem als Eindringsperre für das Gewebe fungierenden inneren Werkzeugführungselement geführt. Zwischen den äußeren und den inneren Werkzeugführungselementen im Bereich der Öffnung für die Applikationswerkzeuge wird das Gewebe fixiert und durch die in entgegengesetzter Richtung wirkenden Applikationswerkzeuge gespannt, und kann - bei begrenzter Eindringtiefe und Eindringgeschwindigkeit - bei maschinell oszillierend wiederholter Penetration auch vibrationsarm penetriert werden. Der Gehäuseinnenraum kann mit einem Tank zur Aufnahme einer zeitgleich oder zeitversetzt zur Penetration in das Gewebe einzubringenden Substanz versehen sein, beispielsweise einer Tätowierflüssigkeit, eines über das Gewebe einzubringenden Impfstoffes oder anderen medizinischen Präparats.

Eine mit einem Gehäuse ausgebildete, manuell betriebene Penetriervorrichtung kann mindestens eine in einem flachen Gehäuse geführte, aus elastischem Material bestehende Ringnadel mit an ihren freien Enden angeformten Applikationswerkzeugen und seitlichen Betätigungsarmen aufweisen. Die Betätigungsarme der Ringnadel ragen aus seitlich gegenüberliegenden Schlitzöffhungen des Gehäuses heraus, die Applikationswerkzeuge durchgreifen eine obere Öffnung des Gehäuses und sind dort an äußeren Werkzeugführungselementen sowie auf eine Gewebeeindringsperre bildenden inneren Werkzeugführungselementen geführt. Darüber hinaus ist die mindestens eine Ringnadel an der den Applikationswerkzeugen gegenüberliegenden Seite an äußeren und inneren Nadelführungselementen geführt. Die jeweils inneren Nadelführungselemente und Werkzeugführungselemente sind durch Stege miteinander verbunden und bilden mit diesen und den Seitenwänden des Gehäuses den Tank für eine möglicherweise auf oder in das Gewebe zu bringende Substanz.

Eine maschinell angetriebene Penetriervorrichtung kann zwei schwenkbar in einem Gehäuse entgegen einer Federwirkung gelagerte und sich kreuzende Betätigungsarme miteinander gegenüberliegenden Betätigungsflächen aufweisen, die zur Erzeugung der Vorspann- und Einstechbewegung mit Betätigungsflächen eines an einem oszillierend beweglichen Betätigungsstößel angebrachten Betätigungskörpers in Wirkverbindung stehen.

Eine maschinell angetriebene Penetriervorrichtung kann zwei in einem Gehäuse parallel zueinander angeordnete, mit Führungsstiften versehene Betätigungsarme und einen in dem Gehäuse entgegen einer Federkraft oszillierend beweglichen Betätigungsstößel mit einem an dessen freiem Ende angebrachten Betätigungskörper aufweisen. Die von den Betätigungsarmen abstrebenden Führungsstifte greifen in derart schräg verlaufende Führungsschlitze des Betätigungskörpers ein, dass die Betätigungsarme mit den an diesen gehaltenen Applikationswerkzeugen bei der Hin- und Herbewegung des Betätigungsstößels in paralleler Ausrichtung abwechselnd aufeinander zu und voneinander weg bewegt werden, um die an den Werkzeugträgern der Betätigungsarme angebrachten Applikationswerkzeuge bzw. Werkzeugkämme in zueinander entgegengesetzter Kraftwirkungsrichtung seitlich in das an der Öffnung des Gehäuses fixierte und durch die Applikationswerkzeuge zunächst vorgespannte Gewebe wiederholt einzutreiben und wieder aus diesem herauszuziehen.

Bei der Penetriervorrichtung können an einer Basisplatte zwei im Abstand und parallel zueinander angeordnete und mittels Führungsbolzen in schräg verlaufenden Führungsschlitzen geführte Betätigungsarme vorgesehen sein, die jeweils an der nach außen weisenden Seite in Längsrichtung der Betätigungsarme schräg verlaufende, durch die Ausbildung einer Nase parallel versetzte Betätigungsflächen aufweisen. Ein die Betätigungsarme seitlich kontaktierender, U-förmiger Betätigungsstößel weist an den Innenseiten seiner Betätigungsschenkel angeformte, ebenfalls durch eine Nase parallel versetzte schräge Betätigungsflächen auf, so dass sich die unter Federwirkung auseinander gedrückten Betätigungsarme bei einer hin und her gehenden Stößelbewegung im Kontakt zwischen den einander kontaktierenden schrägen Betätigungsflächen abwechselnd aufeinander zu oder voneinander weg bewegen und so die an den freien Enden der Betätigungsarme angebrachten, mit den Werkzeugspitzen aufeinander zu gerichteten Applikationswerkzeuge in das beim Penetrieren vorgespannte Gewebe eintreiben bzw. wieder aus diesem herausziehen.

Die Vorrichtung kann drei oder mehr - in Blickrichtung auf eine Anordnungsebene der Werkzeugspitzen - im Winkel zueinander angeordnete und gegeneinander wirkende Applikationswerkzeuge umfassen, deren Werkzeugspitzen vor bzw. hinter einer kreisförmigen, gegebenenfalls bis auf einen Punkt verkleinerten Ansatzlinie auf das Gewebe wirken. In dem Kreis können einander versetzt oder im Winkel α gegenüberliegende Applikationswerkzeuge in zueinander im Wesentlichen entgegengesetzter Richtung und nach Vorspannung des Gewebes in das Gewebe eingreifen.

Die maschinell angetriebene Penetriervorrichtung kann stirnseitig von einem entgegen einer Federkraft hin und her bewegbaren Betätigungsstößel senkrecht abstrebende Applikationswerkzeuge aufweisen, die zwischen einem rotationssymmetrischen, eine konvex gewölbte Mantelfläche aufweisenden inneren Werkzeugführungselement (Gewebeeindringsperre) und einem entsprechend konkav gewölbten, in einer kreisförmigen Öffnung mündenden äußeren Werkzeugführungselement geführt sind. Entlang einer kreisförmigen Anordnungslinie treffen jeweils versetzt gegenüberliegende Applikationswerkzeuge in einem von diesen eingeschlossenen Winkel α im Wesentlichen in entgegengesetzter Kraftrichtung auf das Gewebe, um es zunächst vorzuspannen und anschließend zu penetrieren.

Es kann mindestens eine in einem flachen Gehäuse geführte, manuell betätigbare Ringnadel vorgesehen sein, die seitlich gegenüberliegende Schlitzöffnungen des Gehäuses durchgreifende Betätigungsarme und an ihren freien Enden angeformte, im Bereich der oberen Öffnung des Gehäuses auf inneren Werkzeugführungselementen sowie an äußeren Werkzeugführungselementen geführte Applikationswerkzeuge aufweist.

Die mindestens eine Ringnadel kann an der den Applikationswerkzeugen gegenüberliegenden Seite an äußeren und inneren Nadelführungselementen geführt sein, wobei die jeweils inneren Nadelführungselemente und Werkzeugführungselemente durch Stege verbunden sind und mit diesen und den Seitenwänden des Gehäuses den Tank für den auf oder in das Gewebe aufzubringenden Wirkstoff bilden.

Es können zwei schwenkbar in einem Gehäuse gelagerte und sich kreuzende Betätigungsarme einander gegenüberliegende Betätigungsflächen vorgesehen sein, die mit Betätigungsflächen eines an einem oszillierend bewegten Betätigungsstößel angebrachten Betätigungskörpers in Wirkverbindung stehen.

Auch können zwei in einem Gehäuse parallel zueinander angeordnete, mit Führungsstiften versehene Betätigungsarme und ein in dem Gehäuse oszillierend bewegter Betätigungsstößel mit einem an dessen freiem Ende angebrachten Betätigungskörper vorgesehen sein, wobei die von den Betätigungsarmen abstrebenden Führungsstifte in derart schräg verlaufende Führungsschlitze des Betätigungskörpers eingreifen, dass die Betätigungsarme mit den an diesen gehaltenen Applikationswerkzeugen bei der Hin- und Herbewegung des Betätigungsstößels in paralleler Ausrichtung abwechselnd aufeinander zu und voneinander weg bewegt werden.

Es können zwei im Abstand und parallel zueinander an einer Basisplatte angeordnete und mittels Führungsbolzen in schräg verlaufenden Führungsschlitzen geführte, elastisch auseinander gedrückte Betätigungsarme vorgesehen sein, die jeweils an der nach außen weisenden Seite in Längsrichtung der Betätigungsarme schräg verlaufende Betätigungsflächen aufweisen; sowie einen die Betätigungsarme seitlich kontaktierenden U-förmigen Betätigungsstößel mit an den Innenseiten seiner Betätigungsschenkel angeformten schrägen Betätigungsflächen, so dass sich die federnd auseinander gedrückten Betätigungsarme bei einer Stößelbewegung im Kontakt zwischen den schrägen Betätigungsflächen abwechselnd aufeinander zu oder voneinander weg bewegen.

Es kann vorgesehen sein, dass die Werkzeugenden in der Ruhelage jeweils vor und / oder hinter der Anordnungslinie und / oder unmittelbar auf der Anordnungslinie positioniert sind.

Es kann vorsehen, dass die an einer kreisförmigen Ansatzlinie am Gewebe angreifenden Applikationswerkzeuge senkrecht von der Stirnseite eines an einem Betätigungsstößel gehaltenen, in einem Gehäuseteil geführten kreisförmigen Werkzeugträgers abstreben und zwischen einem rotationssymmetrischen, konvex gewölbten inneren Werkzeugführungselement sowie einem entsprechend konkav gewölbten, eine runde Öffnung aufweisenden äußeren Werkzeugführungselement geführt sind.

### Beschreibung weiterer Ausführungsbeispiele

Im Folgenden werden weitere Ausführungsbeispiele unter Bezugnahme auf Figuren einer Zeichnung näher erläutert. Hierbei zeigen:
- Fig. 1a: eine Draufsicht von zwei entgegengesetzt und zueinander versetzt an einer Anordnungslinie auf ein organisches Gewebe wirkenden Applikationswerkzeugen,
- Fig. 1b: eine Ausführungsvariante der Werkzeuganordnung nach Fig. 1a, bei der ein Applikationswerkzeug durch zwei winklig angeordnete Applikationswerkzeuge ersetzt ist,
- Fig. 2: eine schematische Darstellung nach Fig. 1a zur Definition eines seitlichen Abstandes zwischen zwei gegenüberliegend benachbarten Applikationswerkzeugen,
- Fig. 3a - 3c: Seitenansichten von zwei am organischen Gewebe, insbesondere einer Haut, in unterschiedlicher Winkellage angreifenden Applikationswerkzeugen mit im Abstand versetzt vor / auf / hinter einer gemeinsamen Anordnungslinie versetzt gegenüberliegenden Werkzeugspitzen,
- Fig. 4a: eine perspektivische Darstellung eines manuell betätigten Handgerätes mit mehreren, an mit federnd ausgebildeten Betätigungsarmen gehaltenen Werkzeugträgern gegenüberliegend versetzt zueinander angeordneten Applikationswerkzeugen, wobei ein Vorratsbehälter einer Spendeeinrichtung zwischen den Applikationswerkzeugen angeordnet ist,
- Fig. 4b: eine perspektivische Darstellung eines manuell betätigten Handgerätes mit mehreren, an mit federnd ausgebildeten Betätigungsarmen gehaltenen Werkzeugträgern gegenüberliegend versetzt zueinander angeordneten Applikationswerkzeugen, vergleichbar der Ausführung in Fig. 4a, wobei im Unterschied hierzu ein Vorratsbehälter einer Spendeeinrichtung außerhalb der Applikationswerkzeuge angeordnet ist,
- Fig. 4c: eine perspektivische Darstellung eines manuell betätigten Handgerätes mit mehreren, an mit federnd ausgebildeten Betätigungsarmen gehaltenen Werkzeugträgern gegenüberliegend versetzt zueinander angeordneten Applikationswerkzeugen vergleichbar der Ausführung in Fig. 4b, wobei im Unterschied hierzu die Substanzabgabe auf der Gewebeseite der Werkzeuge erfolgt,
- Fig. 5a - 5: Seitenansichten des in unterschiedlicher Position gemäß Fig. 3 auf das Gewebe aufgesetzten Handgerätes gemäß Fig. 4,
- Fig. 6a - 6d: Ausführungsvarianten von Applikationswerkzeugen mit Wergzeugkämmen als distale Werkzeugenden an einem Basisbauteil jeweils einstückig aus einem elastischen Flachmaterial gefertigt,
- Fig. 7a /: b eine Ausführungsvariante eines Schneidwerkzeuges in Kammform an einem Basisbauteil aus einem elastischen Material gefertigt, in einer Ausgangsstellung (Fig. 7a) und in einer Penetrierstellung (Fig. 7b),
- Fig. 8: eine perspektivische Darstellung eines manuell betätigten Handgerätes mit einem in einem Gehäuse geführten, elastisch verformbaren ringförmigen Werkzeugträger,
- Fig. 9: eine Innenansicht einer Gehäusehälfte der Vorrichtung nach Fig. 8,
- Fig. 10: ein Funktionsschema der in Fig. 8 und 9 gezeigten Vorrichtung mit einer einstellbaren Gewebeeindringsperre,
- Fig. 11a/b: eine Ausführungsform eines Betätigungsmechanismus für die Applikationswerkzeuge mit einem von einem maschinell angetriebenen Stößel betätigten Werkzeugträger in einer Position vor dem Einstechen (Ausgangsstellung) (Fig. 11a) und nach dem Einstechen (Penetrierstellung) (Fig. 11b) in das Gewebe,
- Fig. 12a / b: eine weitere Ausführungsform eines maschinell angetriebenen Handgerätes in einer Position vor dem Einstechen (Ausgangsstellung) (Fig. 12a) und in einer Position nach dem Einstechen (Penetrierstellung) (Fig. 12b),
- Fig. 13a - 13d: eine andere Ausführungsform einer maschinell betriebenen Vorrichtung in einer Position vor dem Einstechen (Ausgangsstellung) und nach dem Einstechen (Penetrierstellung) der Applikationswerkzeuge,
- Fig. 14: eine Außenansicht einer geometrischen Ausgestaltungsvariante eines Gehäuses einer Vorrichtung zum Applizieren eines Wirkstoffen auf oder in ein organisches Gewebe gemäß Fig. 12, 13 oder 15 mit Spendeeinrichtung für den zu applizierenden Wirkstoff,
- Fig. 15a/b: eine Ausführungsform einer maschinell betriebenen Vorrichtung mit an einer kreisförmigen Ansatzlinie auf das Gewebe wirkenden winklig zueinander angeordneten Applikationswerkzeugen in einer Position vor dem Einstechen (Fig. 15a) und in einer Position nach dem Einstechen (Fig. 15b) und
- Fig. 16: eine schematische Darstellung der Führung distaler Werkzeugenden in zugeordneten Aufnahmen.

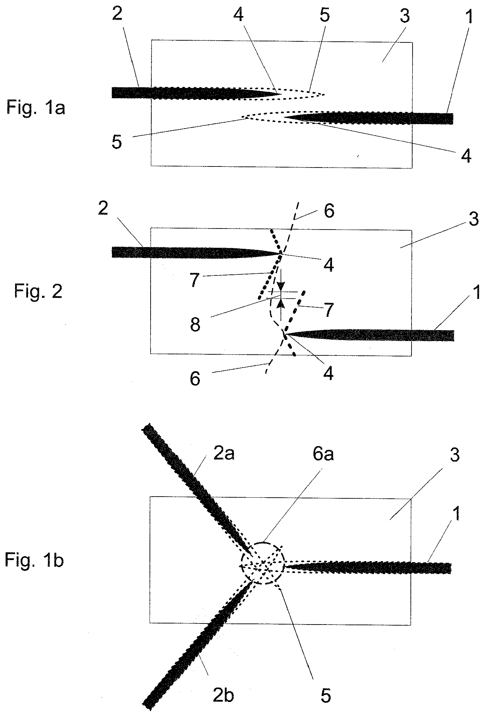

Fig. 1 bis 3 zeigen eine Draufsicht verschiedener Ausführungsvarianten von Werkzeuganordnungen einer Vorrichtung zum Penetrieren eines organischen Gewebes und Applizieren eines Wirkstoffs auf oder in ein organisches Gewebe, wonach mindestens zwei sich paarweise, jedoch in geringem Abstand versetzt zueinander gegenüberstehende und bei der Penetration in einem Winkel α mit entgegengesetzter Kraftwirkungsrichtung aufeinander zu bewegbare spitze und / oder scharfe, stechende und / oder schneidende - erste und zweite - Applikationswerkzeuge 1, 2 nach dem Aufsetzen auf das Gewebe 3, beispielsweise die Haut, in der ersten Bewegungsphase das im Wirkungsbereich von Spitzen von Werkzeugenden 4 (distalen Enden) liegende Gewebe zunächst vorspannen und anschließend mit aufgrund der automatischen Vorspannung möglicher, aber nicht notwendig geringer Geschwindigkeit flach in das Gewebe eindringen und jeweils einen Penetrierkanal 5 oder -schlitz erzeugen. Die Spitzen der Werkzeugenden 4 sind auf einer Gewebeoberfläche 3a in einem Applikationsbereich 3b angeordnet.

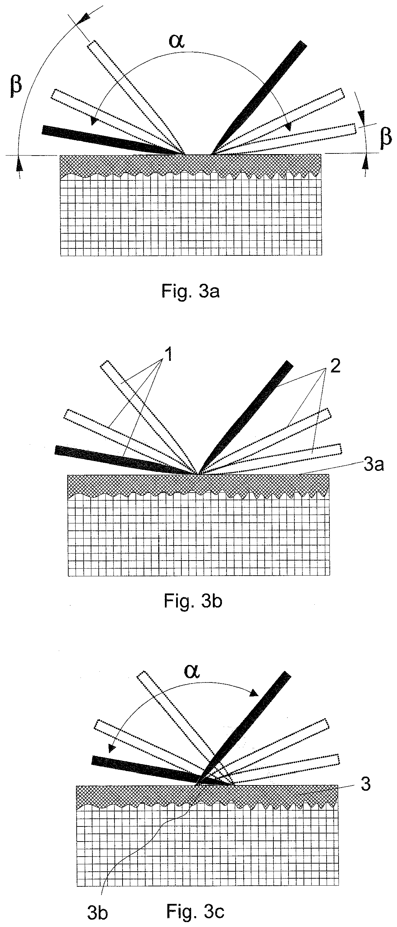

In der schematischen Darstellung gemäß Fig. 1a, 2 und 3b liegen die Spitzen der Werkzeugenden 4 mit der Penetriereinrichtung der beiden Applikationswerkzeuge 1, 2 beim Aufsetzen auf das Gewebe 3 auf einer Höhe, d.h. auf einer gemeinsamen Anordnungslinie 6. Die Spitzen der Werkzeugenden 4 können in der Ausgangslage jedoch auch jeweils vor (Fig. 3a) oder jeweils hinter dieser gemeinsamen Anordnungslinie 6 (Fig. 3c) verlaufen. Der seitliche Abstand zwischen zwei benachbarten (gegenüberliegenden) Werkzeugspitzen 4 ist, wie Fig. 2 zeigt, so eng gewählt, dass benachbarte Wirkungsbereiche 7 der Werkzeugspitzen 4 einen gemeinsamen Überschneidungsbereich 8 bilden. Dadurch ist gewährleistet, dass das Gewebe aufgrund der entgegengesetzt gerichteten Kraftwirkung benachbarter Werkzeugspitzen 4 vor dem Einstechen vorgespannt wird.

Wie die Fig. 3a bis 3c zeigen, können die entgegengesetzt gerichteten Applikationswerkzeuge 1, 2 in jeweils unterschiedlichem spitzen Winkel β oder - wie mit den strichlierten Linien angedeutet - auch in einem übereinstimmenden Winkel zur Gewebeoberfläche ausgerichtet sein. Der zwischen den gegenüberliegenden, entgegengesetzt gerichteten Applikationswerkzeugen 1, 2 eingeschlossene Winkel α in der Projektionsebene quer zur Wirkrichtung der Applikationswerkzeuge ist, wie die Fig. 3a bis 3b zeigen, kleiner als 180° und größer als 0° und liegt, wie die in der Zeichnung dem Gewebe 3 jeweils am nächsten liegenden Applikationswerkzeuge 1, 2 zeigen, vorzugsweise zwischen 150° und 130°. Durch die Winkel α/β wird die Eindringtiefe der Applikationswerkzeuge in das Gewebe maßgeblich bestimmt.

In den Fig. 1a, 2 und 3a bis 3b ist zur Erläuterung eines bei mehreren Ausführungen auftretenden Vorspannprinzips jeweils nur ein Paar gegenüberliegender und seitlich zueinander versetzter Applikationswerkzeuge 1, 2 dargestellt. Fig. 1b zeigt eine erweiterte Ausführungsform eines Werkzeugpaars, bei der in Draufsicht dem ersten Applikationswerkzeug 1 zwei im Winkel zueinander angeordnete zweite Applikationswerkzeuge 2a, 2b gegenüberstehen. In diesem Fall sind die Spitzen von Werkzeugenden 4 von drei zueinander versetzt auf das Gewebe wirkenden Applikationswerkzeugen 1, 2a, 2b auf dem Gewebe an einer kreisförmigen bis punktförmigen Ansatzlinie 6a oder sich überkreuzend (nicht dargestellt) hinter dieser Ansatzlinie 6a angeordnet.

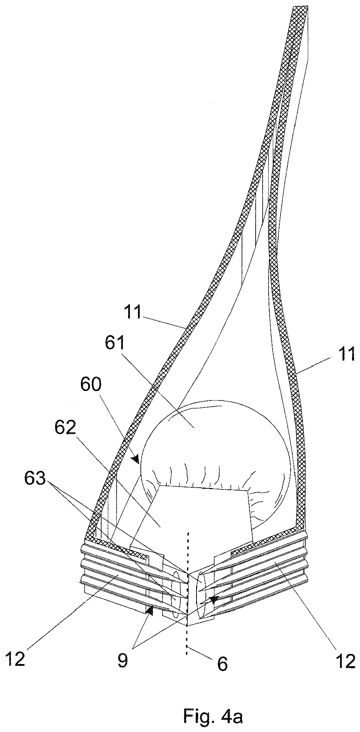

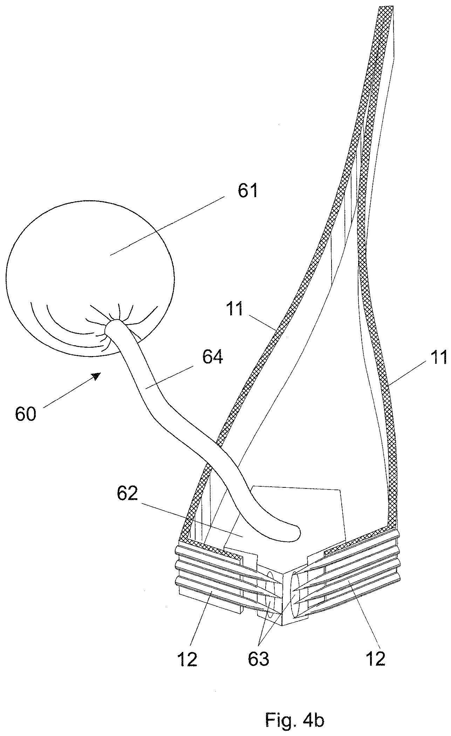

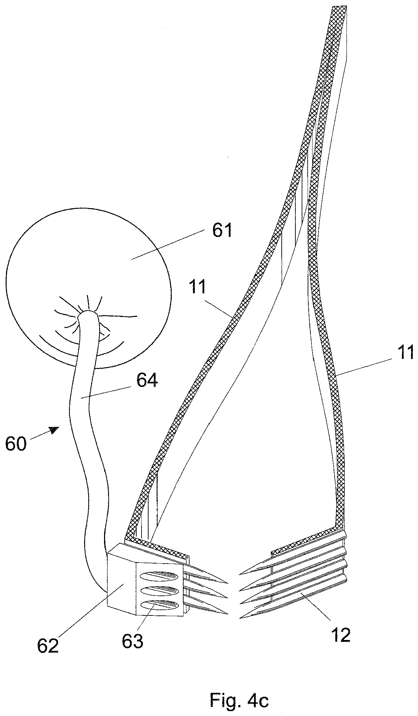

Wie die Fig. 4, 6, 7a und 7b zeigen, befinden sich bei dem dargestellten Ausführungsbeispiel auf jeder Seite der Anordnungslinie 6 mehrere zueinander versetzt gegenüberliegende Applikationswerkzeuge 1 und 2, die jeweils einen Werkzeugkamm 9 bilden. Die gemeinsame Anordnungslinie 6, an der die gegenüberliegenden Applikationswerkzeuge 1, 2 oder die Werkzeugkämme 9 ausgerichtet sind, kann gerade oder gekrümmt sein oder auch zwei oder mehrere im Winkel angeordnete Geraden umfassen. Es ist darüber hinaus auch denkbar, dass nur auf einer Seite ein Applikationswerkzeug bzw. Werkzeugkamm angeordnet ist und auf der gegenüberliegenden Seite nur ein das Gewebe vorspannendes Haltewerkzeug (nicht dargestellt) oder Werkzeugkamm ohne penetrierende Werkzeugenden vorgesehen ist.

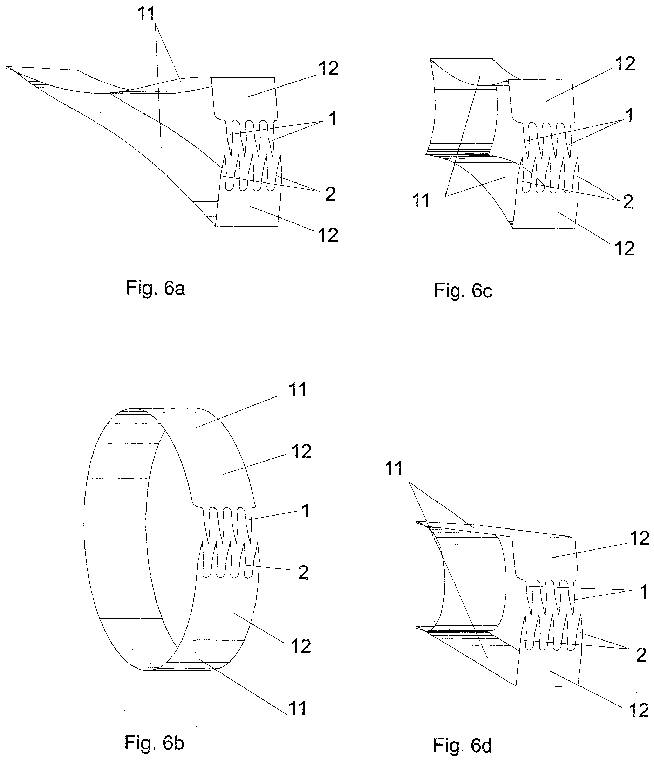

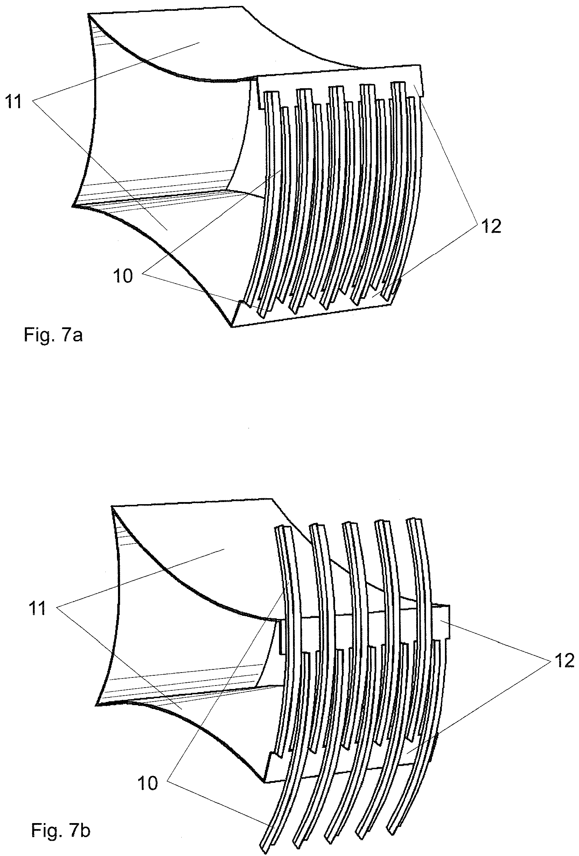

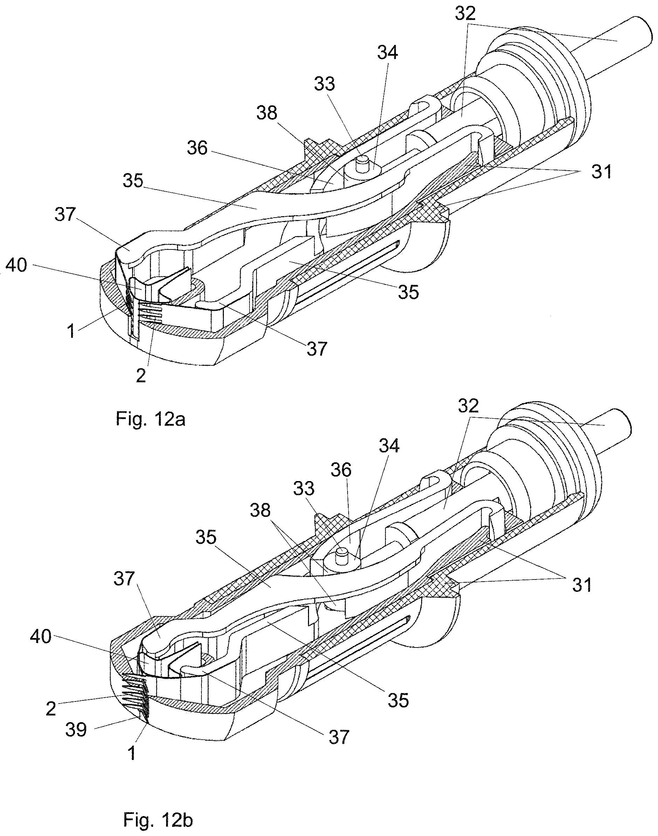

Die Applikationswerkzeuge 1, 2 sind gemäß der Darstellung in den Fig. 1 bis 3 sowie den weiter unten erläuterten Fig. 4, 5, 8, 9, 13 und 15 als spitze Nadeln mit rundem Querschnitt ausgebildet. Die Applikationswerkzeuge 1, 2 können aber auch Hohlnadeln / Kanülen (nicht dargestellt) sein oder einen flachen Querschnitt mit einer Spitze (Fig. 6a-d, Fig. 12a-b) oder einer scharfen Schneide aufweisen oder gemäß der Darstellung in den Fig. 7a und 7b als in Längsrichtung verlaufende Messer oder Schneiden 10 zur Penetrierung des Gewebes, insbesondere der Haut mittels Längsschlitzen ausgeführt sein oder als einseitig stumpfe distale Werkzeugenden ausgebildet sein.

Ein wesentliches Anwendungsgebiet der Vorrichtung zum Penetrieren mit der anhand der Fig. 1 bis 3 erläuterten Werkzeuganordnung ist die Penetration von organischem Gewebe zum zeitgleichen oder zeitversetzten Einbringen bestimmter kosmetischer oder medizinischer Substanzen in oder auf das Gewebe oder über das Gewebe in den Körper bzw. ein Organ.

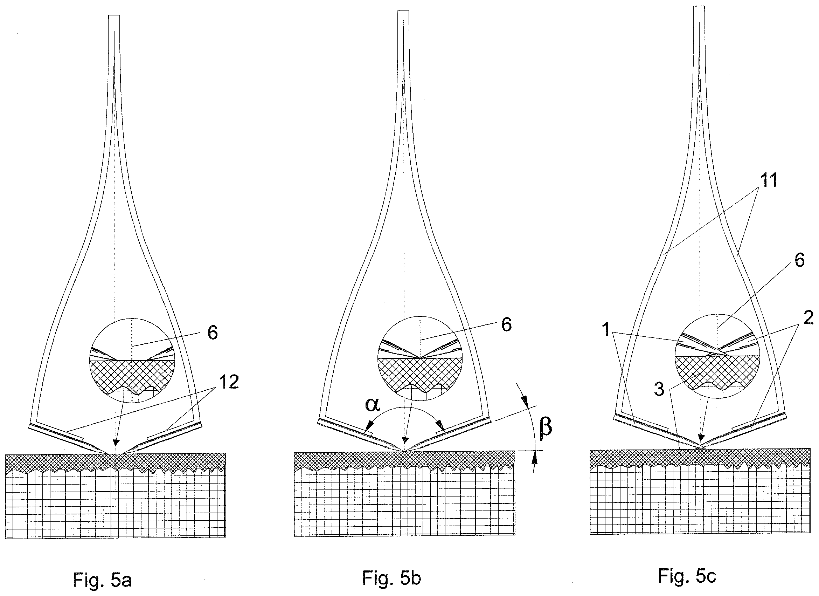

Die in den Fig. 4a bis 4c und 5 gezeigte manuell betätigbare Vorrichtung umfasst zwei miteinander verbundene, elastisch federnde Betätigungsarme 11 mit von deren freien Enden auskragenden Werkzeugträgern 12 und an diesen befestigten, hier als spitze Nadeln ausgebildeten und jeweils einen Werkzeugkamm 9 bildenden Applikationswerkzeugen 1, 2. Nach dem Aufsetzen der Spitzen der Werkzeugenden 4 (distalen Werkzeugenden) auf das Gewebe werden die Betätigungsarme 11 mit den Fingern einer Hand zusammengedrückt und dabei die Applikationswerkzeuge 1, 2 in entgegengesetzter Richtung unter gleichzeitiger Vorspannung des Gewebes in einem spitzen (flachen) Winkel β und einer diesem entsprechenden Eindringtiefe in das Gewebe eingetrieben. Nach dem Freigeben der Betätigungsarme 11 federn die Applikationswerkzeuge 1, 2 oder Werkzeugkämme 9 in die Ausgangslage zurück und der Vorgang kann beliebig oft wiederholt werden.

Eine Spendeeinrichtung 60 zum Ausbringen des zu applizierenden Wirkstoffes kann beispielsweise zwischen den Betätigungsarmen 11 angeordnet sein (vgl. Fig. 4a), so dass mittels Betätigen der Betätigungsarme 11 der Wirkstoff, welcher in einem Vorratsbehälter 61 zum Ausbringen bereitgestellt ist, freigegeben wird. Der Vorratsbehälter 61 steht in Fluidverbindung 64 mit einem Spendebauteil 62, welches Öffnungen 63 zum Ausbringen des Wirkstoffes hin zu den Applikationswerkzeugen aufweist. So kann beispielsweise ein Medikament über die Haut oder zum Einbringen einer Tätowierflüssigkeit in die obere Hautschicht verabreicht werden. Die Spendeeinrichtung 60, zumindest der Vorratsbehälter 61, kann aber ebenso außerhalb der Betätigungsarme 11 angebracht sein (vgl. Fig. 4b). Es kann in einer Ausgestaltung vorgesehen sein (vgl. Fig. 4c), dass der Wirkstoff entlang des Werkzeugträgers 12 zum Penetrierbereich hin läuft.

Die Fig. 6a bis 6d zeigen Ausführungsvarianten von Applikationswerkzeugen mit Werkzeugkämmen als distale Werkzeugenden an einem Basisbauteil jeweils einstückig aus einem V-förmig, ringförmig oder trapezförmig gestalteten elastischem Flachmaterial, zum Beispiel einem Blechstreifen, gefertigt, der sowohl die Betätigungsarme 11, den Werkzeugträger 12 und die Applikationswerkzeuge 1, 2 oder die beiden Werkzeugkämme bildet und von denen mindestens ein Werkzeugkamm zum Penetrieren des organischen Gewebes befähigt ist. Diese einstückigen Applikationsvorrichtungen können entweder manuell betätigt werden oder in eine maschinell betriebene Vorrichtung integriert sein.

Die in Fig. 7a und 7b (in Ausgangslage und in Penetrierstellung) dargestellte Vorrichtung ist ebenfalls mit einem elastischen Blechstreifen hergestellt. In diesem Fall sind jedoch die an die Werkzeugträger 12 angeformten Applikationswerkzeuge als Messer 10 zum Öffnen der Hautoberfläche mittels Schlitzen, beispielsweise zur zeitgleichen oder zeitversetzten Applikation einer Substanz in die Haut oder das Gewebe, ausgebildet.

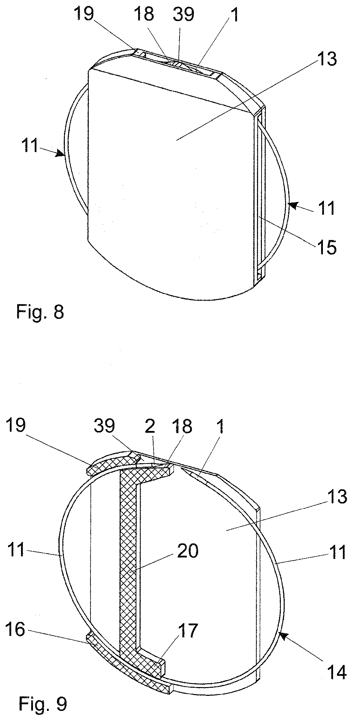

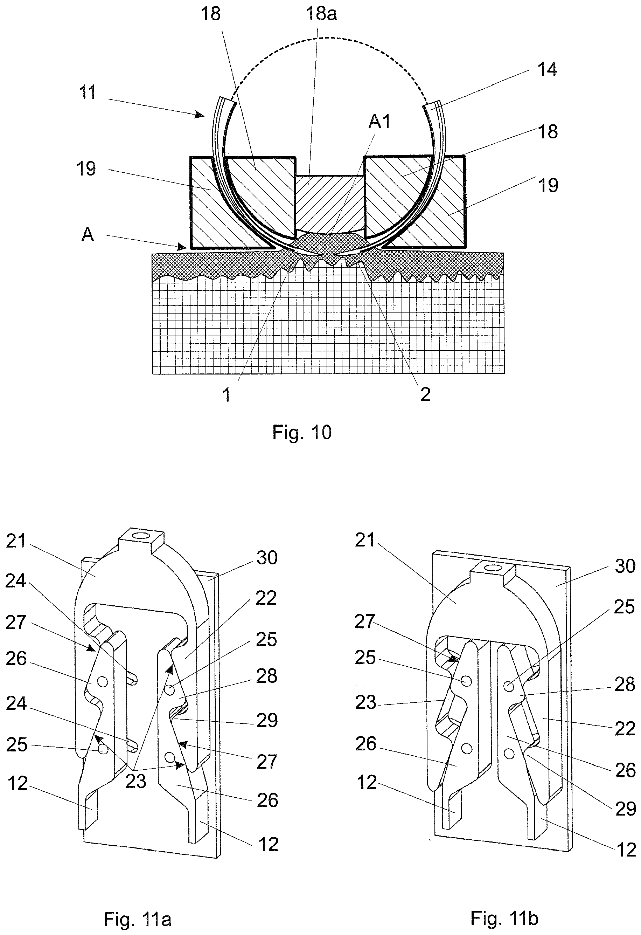

Die Fig. 8 und 9 zeigen eine Ausführungsvariante einer manuell betriebenen, mit einem Gehäuse 13 versehenen Vorrichtung, die als elastische, offene Ringnadel 14 mit durch seitliche als Schlitzöffnungen ausgeführte Öffnungen 15 des Gehäuses 13 ragenden seitlichen Betätigungsarmen 11 und an deren freien Enden angeformten Applikationswerkzeugen 1, 2 ausgeführt ist. Die Ringnadel 14 ist an der den Applikationswerkzeugen 1, 2 gegenüberliegenden Seite zwischen äußeren und inneren Nadelführungselementen 16, 17 geführt. Die Applikationswerkzeuge 1, 2 der Ringnadel 14 sind zwischen inneren Werkzeugführungselementen 18 und - unter Bildung einer Öffnung 39 im Gehäuse 13 - im Abstand voneinander angeordneten äußeren Werkzeugführungselementen 19 gehalten. Bei der Benutzung dieser Penetriervorrichtung stützen sich, wie Fig. 10 schematisch zeigt, die beiden äußeren Werkzeugführungselemente 19 auf der Gewebeoberfläche A ab, während der zwischen diesen liegende Gewebebereich A1 an dem weiter innen liegenden und gleichzeitig als Gewebeeindringsperre (18a in Fig. 10) fungierenden inneren Werkzeugführungselementen 18 gehalten ist. An oder in dem Gehäuse befindet sich ein Vorratstank (nicht dargestellt) für den oder die zu applizierenden Wirkstoffe, der durch Betätigung der Applikationswerkzeuge den zu applizierenden Wirkstoff freigibt, in der Art, dass der Wirkstoff in den Penetrierbereich (in den Bereich der Öffnung 5) gelangt. Die Ringnadel kann aber auch als Ringhohlnadel ausgebildet sein und in diesem Fall direkt mit der Spendeeinrichtung verbunden sein, so dass bei Betätigen der Vorrichtung der zu applizierende Wirkstoff über die Ringhohlnadel ausgebracht wird.

Die in Fig. 10 schematisch dargestellte Gewebeeindringsperre 18a kann einstellbar angeordnet sein, um die Eindringtiefe des Gewebes in die Öffnung und somit die vertikale Einstechtiefe der Applikationswerkzeuge 1, 2 in das Gewebe einstellen zu können. Bei der Betätigung der Vorrichtung an den beiden seitlichen, über die Schlitze 15 aus dem Gehäuse 13 herausragenden Betätigungsarmen 11 wird das Gewebe zwischen den äußeren Werkzeugführungselementen 19 und auf den inneren Werkzeugführungselementen 18 (Gewebeeindringsperre 18a) fixiert und durch die zueinander versetzt in entgegengesetzter Richtung auf das Gewebe wirkenden Applikationswerkzeuge 1, 2 vorgespannt, so dass die Applikationswerkzeuge bei geringer vertikaler Einstechtiefe in das Gewebe eindringen können.

In Fig. 9 ist der Einfachheit halber jeweils nur ein äußeres und inneres Werkzeugführungselement bzw. Nadelführungselement 16 bis 19 dargestellt, die korrespondierenden bzw. ergänzenden Strukturen werden von der herstellungstechnisch vorteilhafterweise identischen und nicht dargestellten Gehäusehälfte beigesteuert. Die jeweils inneren Werkzeug- und Nadelführungselemente 17, 18 können jeweils über einen Steg 20 verbunden sein, um so zusammen mit den Innenflächen des Gehäuses 13 einen kleinen Tank, der dann Teil der Spendeeinrichtung 60 ist, zur Aufnahme eines Tätowiermittels, eines pharmazeutischen Präparats oder dergleichen, zu bilden. Bei Verwendung einer massiven Ringnadel 14 kann über eine kleine Öffnung zwischen den beiden inneren Werkzeugführungselementen 18 ein Impfstoff, ein Tätowiermittel oder dergleichen aus dem Tank vor oder während der Behandlung austreten und nach oder mit dem Stechvorgang in das Gewebe eindringen. Zudem kann bei der Verwendung von Hohlnadeln als Ringnadel 14 die Anbindung der Nadellumina an den Tank im Bereich der korrespondierenden inneren Nadelführungselemente 17 erfolgen.

Die Fig. 11a und 11b zeigen eine Ausführungsform eines Betätigungsmechanismus für die Applikationswerkzeuge in einer Position vor und nach dem Eindringen der - hier nicht dargestellten - Applikationswerkzeuge. An einer Basisplatte 30 ist als Betätigungselement ein maschinell angetriebener, etwa U-förmiger Betätigungsstößel 21, der an den Innenseiten der beiden Betätigungsschenkel 22 jeweils zwei erste abgeschrägte, durch Nasen 29 parallel versetzte Betätigungsflächen 23 aufweist, auf und ab bewegbar. Die Basisplatte 30 weist zwei Paar schräg nach unten gerichtete Führungsschlitze 24 auf, in die jeweils Führungsbolzen 25 von zwei im Abstand parallel zueinander angeordneten Betätigungsarmen 26 eingreifen. Jeweils ein am unteren Ende der Betätigungsarme 26 vorgesehener Werkzeugträger 12 dient zur Befestigung von in einem vorgegebenen Winkel α zueinander entgegengesetzt gerichteten, zueinander versetzten Applikationswerkzeugen oder Werkzeugkämmen (nicht dargestellt).

Die schrägen Betätigungsflächen 23 an den Betätigungsschenkeln 22 stehen mit in gleicher Richtung verlaufenden zweiten schrägen und über Nasen 28 parallel versetzten Betätigungsflächen 27 der Betätigungsarme 26 in Wirkverbindung. Fig. 11a zeigt den Betätigungsstößel 21 in einer oberen Position und die beiden Betätigungsarme 26 in einer - mithilfe einer zwischen beiden Betätigungsarme 26 angeordneten Druckfeder (nicht dargestellt) - auseinandergezogenen Position, in der die Applikationswerkzeuge (in Fig. 11a/b nicht dargestellt) nicht in das Gewebe eingreifen. In der in Fig. 11b wiedergegebenen Darstellung befindet sich der Betätigungsstößel 21 in der unteren Position, wobei die Betätigungsarme 26 während der Abwärtsbewegung des Betätigungsstößels 21 entlang der Führungsschlitze 24 aufeinander zu bewegt wurden und die Applikationswerkzeuge somit in einem spitzen Winkel β zur Hautoberfläche in das Gewebe eindringen können.

Eine in den Fig. 12a und 12b gezeigte Ausführungsvariante einer maschinell angetriebenen Vorrichtung umfasst einen innerhalb eines zweiteiligen Gehäuses 31 maschinell hin und her bewegbaren Betätigungsstößel 32 mit einem an dessen freiem Ende angebrachten, mittels Führungsstift 33 in Führungsschlitzen (nicht dargestellt) des Gehäuses 31 geführten Betätigungskörper 34. In dem Gehäuse 31 sind zwei sich kreuzende und schwenkbare Betätigungsarme 35 mit jeweils an deren Innenseite ausgebildeter - konkav gewölbter - Betätigungsfläche 36 und am freien Enden ausgebildetem Werkzeugträger 37 angeordnet. Eine Betätigungsfläche 38 des Betätigungskörpers 34 steht mit den an den Innenseiten der Betätigungsarme 35 vorgesehenen gewölbten Betätigungsflächen 36 in Wirkverbindung. An den Werkzeugträgern 37 der Betätigungsarme 35 sind entgegengesetzt gerichtete, zueinander versetzte, hier jeweils als Werkzeugkamm ausgeführte Applikationswerkzeuge 1, 2 angebracht, die an einem im Innern des Gehäuses 31 unterhalb einer Schlitzöffnung 39 vorgesehenen inneren Werkzeugführungselement 40 (Gewebeeindringsperre) geführt sind. In dem Werkzeugführungselement 40 können Führungsnuten (nicht dargestellt) zur Führung der Applikationswerkzeuge 1, 2 ausgebildet sein.

Beim Aufsetzen der Vorrichtung auf das Gewebe wirkt das Werkzeugführungselement 40 als das Gewebe gemäß Fig. 10 in senkrechter Richtung fixierende Gewebeeindringsperre 18a (Fig. 10). Bei der Vorwärtsbewegung des Betätigungskörpers 34 entlang der gekrümmten Betätigungsflächen 36 der Betätigungsarme 35 werden die Applikationswerkzeuge 1, 2 entgegen der Federkraft einer zwischen den beiden Betätigungsarmen vorgesehenen Druckfeder (nicht dargestellt) aufeinander zu bewegt, um das Gewebe und insbesondere die Haut zunächst vorzuspannen und dann in dieses bzw. diese einzudringen. Bei der Rückbewegung des Betätigungskörpers 34 werden die Betätigungsarme 35 aufgrund der Federkraft wieder in ihre Ausgangslage zurück verschwenkt und dabei die Applikationswerkzeuge wieder aus dem Gewebe herausgezogen.

Das in den Fig. 12a und 12b gezeigte Modul kann an ein Antriebsmodul (nicht dargestellt) angekoppelt werden, zum Beispiel mittels Aufstecken und / oder Anschrauben. Derartige Antriebsmodule sind als solche in verschiedenen Ausführungen bekannt, zum Beispiel in Verbindung mit Handgeräten zum Tätowieren oder zum Ausbilden von permanentem Make-up. Üblicherweise stellt das Antriebsmodul eine lineare Vor- und Rückwärtsbewegung mit einer Betriebsfrequenz zur Verfügung, die bei der Ausführung in den Fig. 12a und 12b auf den vor und zurück bewegbaren Betätigungsstößel 32 eingekoppelt wird.

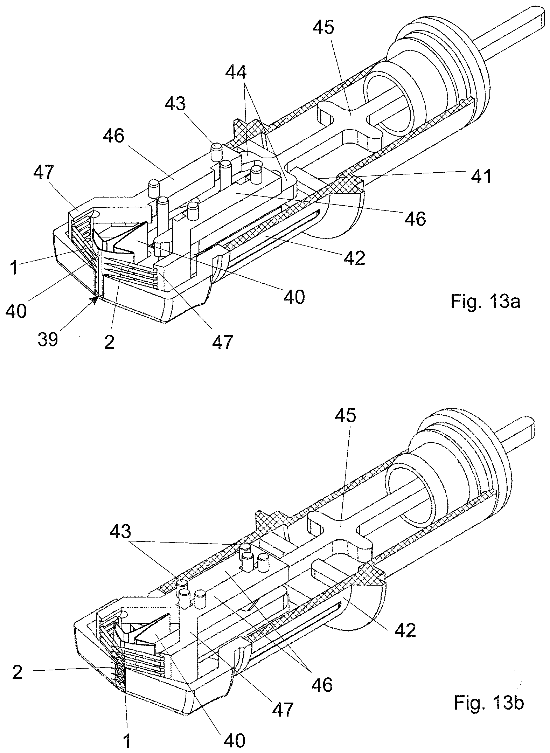

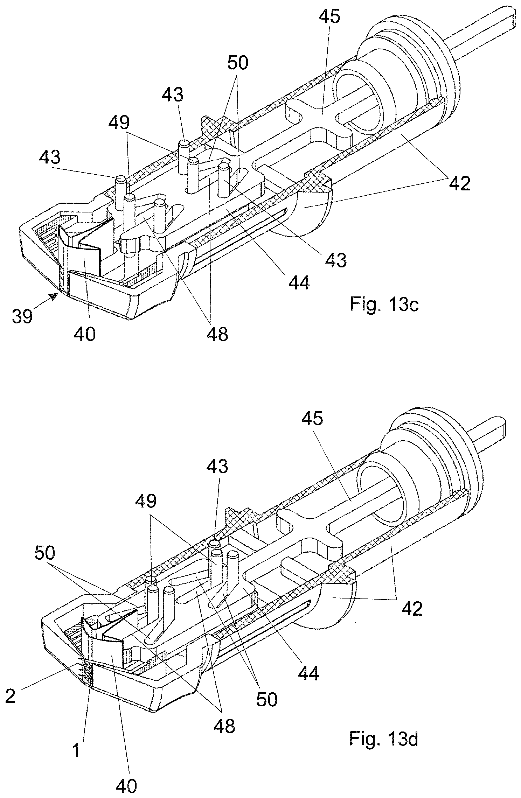

Eine weitere Ausführung einer maschinell angetriebenen Vorrichtung zum wiederholten Penetration eines Gewebes, zum Beispiel zum Applikation eines Wirkstoffes, wird anhand der Fig. 13a, 13b, 13c und 13d erläutert. Das gezeigte Modul ist, vergleichbar der Ausführung in den Fig. 12a und 12b, lösbar an eine Antriebseinrichtung (nicht dargestellt) koppelbar.

Wie die Fig. 13a und 13b zeigen, sind in einem Gehäuse 42 zwei über Führungsstifte 43 mit einem Betätigungskörper 44 eines maschinell angetriebenen Betätigungsstößels 45 in Wirkverbindung stehende Betätigungsarme 46 beweglich gelagert. An einem an den vorderen Enden der Betätigungsarme 46 ausgebildeten Werkzeugträger 47 sind die - hier nadelförmig gestalteten und an einem gleichzeitig als Gewebeeindringsperre fungierenden Werkzeugführungselement 40 geführten - Applikationswerkzeuge 1, 2 befestigt. Die in Fig. 13a in einer Ausgangsstellung vor dem Penetrieren gezeigten Applikationswerkzeuge 1, 2 werden, wie in Fig. 13b dargestellt, bei der Vorwärtsbewegung des Betätigungsstößels 45 und der dadurch bewirkten Verschiebung der Betätigungsarme 46 in entgegengesetzter Richtung aufeinander zu bewegt, um die Öffnung 39 zu durchgreifen und das Gewebe bei aufgesetzter Penetriervorrichtung penetrieren zu können.

Aus den Fig. 13c und 13d ist die Funktion des mit dem Betätigungsstößel 45 verbundenen Betätigungskörpers 44 zur Bewegung der Betätigungsarme 46 und damit der Applikationswerkzeuge 1, 2 ersichtlich. Der Betätigungskörper 44 weist zum einen in Stößelbewegungsrichtung verlaufende erste gerade Führungsschlitze 48 auf, in die am Gehäuse 42 angebrachte Führungsstifte 49 eingreifen, um den Betätigungskörper 44 linear zu führen. In die darüber hinaus im Betätigungskörper 44 ausgebildeten zweiten, schräg verlaufenden Führungsschlitze 50 greifen die an den Betätigungsarmen 46 befestigten Führungsstifte 43 ein, so dass die Betätigungsarme 46 bei der Vorwärtsbewegung des Betätigungsstößel 45 aus der in Fig. 13c gezeigten Stellung in eine in Fig. 13d gezeigte Position aufeinander zu nach innen bewegt werden und damit die Applikationswerkzeuge 1, 2 in einer sich kreuzenden Bewegung durch die Öffnung 39 hindurch in das Gewebe eingreifen können. Bei der Rückwärtsbewegung des Betätigungsstößels 45 werden die Betätigungsarme 46 wieder nach außen verschoben und damit die Applikationswerkzeuge 1, 2 zurückgezogen. Ein am Betätigungsstößel 45 angeformter erster Haltesteg 41 dient zur Anlage einer zwischen diesem und einem weiteren, am Gehäuse angeformten Haltesteg 41b vorgesehenen Druckfeder (nicht dargestellt), die den Betätigungsstößel 45 durch Federkraft in die Ausgangslage verschiebt.

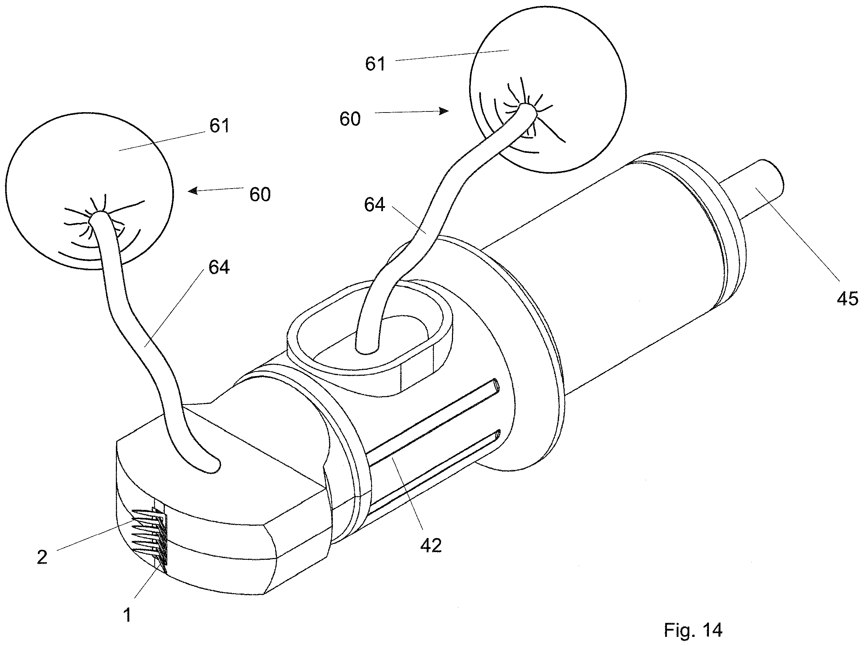

Fig. 14 zeigt eine geometrische Ausgestaltungsvariante eines Gehäuses 42 einer Vorrichtung zum Applizieren eines Wirkstoffs auf oder in ein organisches Gewebe gemäß Fig. 12, 13 oder 15 mit einer schematisch dargestellten (doppelten) Spendeeinrichtung 60 für den zu applizierenden Wirkstoff, mit einer Öffnung für die mindestens zwei Applikationswerkzeuge 1, 2 sowie für das Ausbringen des Wirkstoffs und mit einem Betätigungsstößel 45. Hierbei kann auch nur eine der in Fig. 14 gezeigten Spendeeinrichtungen 60 vorgesehen sein, bei denen im gezeigten Beispiel jeweils der Vorratsbehälter 61 über eine Fluidleitung 64 ans Gehäuse 42 koppelt, in welchem dann der ausgebrachte Wirkstoff hin zu den Applikationswerkzeugen 1, 2 geführt wird. Der Betätigungsstößel 45 kann beispielsweise an eine handelsübliche motorisierte Tätowiermaschine gekoppelt werden, so dass das in Fig. 14 gezeigte Bauteil als wechselbarer Tätowier- oder Stechaufsatz (Nadelmodul) dient. In dieser oder anderen Ausführungen kann das Nadelmodul mit den Applikationswerkzeugen als Einwegartikel ausgeführt sein, welcher nach der Nutzung entsorgt wird.

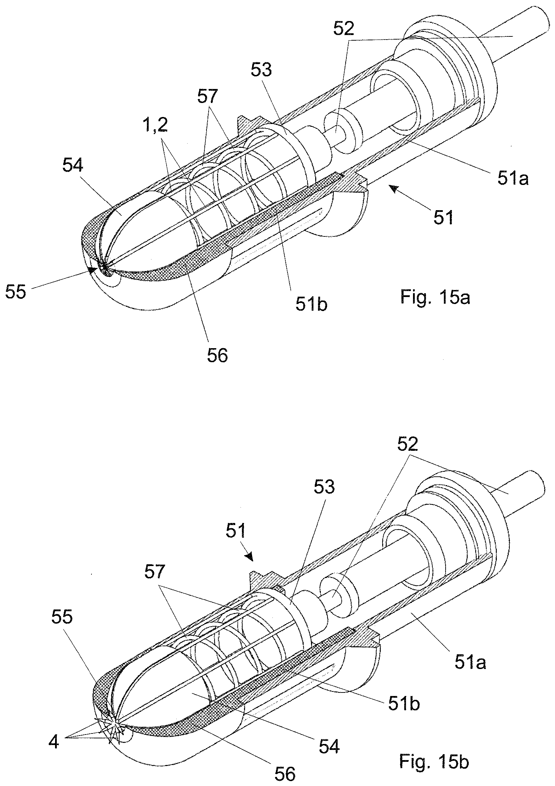

Eine in den Fig. 15a und 15b gezeigte weitere Ausführungsvariante einer maschinell angetriebenen Vorrichtung, die als Handgerät ausgeführt ist, umfasst einen in einem zweiteiligen zylindrischen, aus einem ersten und einem zweiten Gehäuseteil 51a, 51b bestehenden Gehäuse 51 hin und her bewegbaren Betätigungsstößel 52, die in einer alternativen Ausgestaltung auch manuell bedienbar ausgeführt sein kann.

Von einem an dem Betätigungsstößel 52 angebrachten, in dem Gehäuseteil 51b geführten kreisförmigen Werkzeugträger 53 strebt eine Mehrzahl von kreisförmig angeordneten Applikationswerkzeugen 1, 2 ab, die (vorzugsweise in - hier nicht dargestellten - Führungsnuten) zwischen einem rotationssymmetrischen, eine konvex gewölbte Mantelfläche aufweisenden inneren Werkzeugführungselement 54 und einem entsprechend konkav gewölbten, in einer kreisförmigen Öffnung 55 mündenden äußeren Werkzeugführungselement 56 geführt sind. Zwischen dem Werkzeugträger 53 und dem inneren Werkzeugführungselement 54 ist eine Druckfeder 57 angeordnet. In der in Fig. 15a gezeigten Ausgangsstellung sind die Werkzeugspitzen 4 im Bereich der runden Öffnung 55 entlang einer kreisförmigen Ansatzlinie positioniert. Bei der Vorwärtsbewegung des Betätigungsstößels 52 entgegen der Federkraft der Druckfeder 57 durchdringen die Werkzeugspitzen 4 der Applikationswerkzeuge 1, 2 die kreisförmige Öffnung 55, wobei eine Mehrzahl von versetzt gegenüberliegenden und jeweils in entgegengesetzter Richtung wirkenden - kreisförmig angeordneten - jeweils einen Winkel α einschließenden Applikationswerkzeugpaaren das Gewebe zunächst vorspannt und dann in das Gewebe eindringt. Zeitgleich oder zeitversetzt mit dem Einstechen in das Gewebe kann ein Wirkstoff, beispielsweise ein Tätowiermittel oder eine andere Substanz, in das Gewebe eingebracht werden.

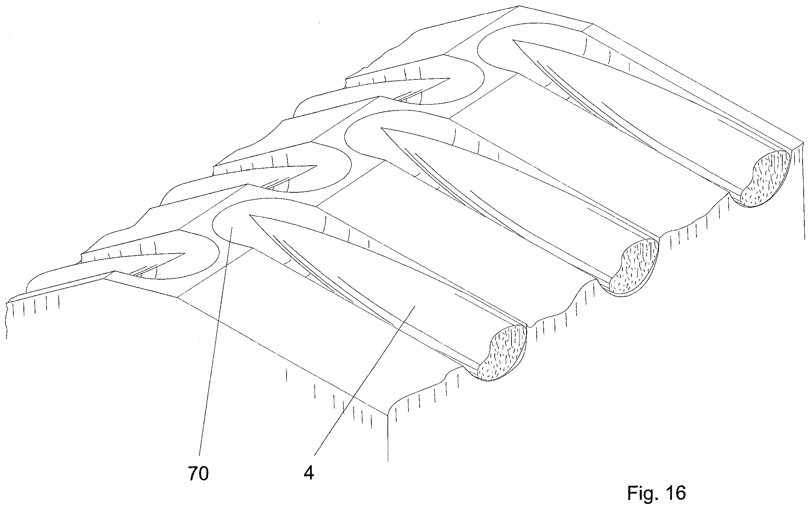

Fig. 16 zeigt eine schematische Darstellung der mechanischen Führung der Werkzeugenden 4 von mehreren Applikationswerkzeugen in einer jeweils zugeordneten Aufnahme 70.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

### Bezugszeichenliste

- 1: erstes Applikationswerkzeug
- 2: zweites Applikationswerkzeug (2a, 2b)
- 3: Gewebe (3a Gewebeoberfläche, 3b Applikationsbereich)
- 4: Werkzeugenden
- 5: Penetrierkanal im Gewebe
- 6: Anordnungslinie /Ansatzlinie 6a der Werkzeugspitzen
- 7: Wirkungsbereich / Hautverschiebung von 4
- 8: Überschneidungsbereich von 7
- 9: Werkzeugkamm
- 10: Schneidklinge
- 11: Betätigungsarm
- 12: Werkzeugträger
- 13: Gehäuse
- 14: Ringnadel
- 15: seitliche Öffnungen in 13
- 16: äußeres Nadelführungselement
- 17: inneres Nadelführungselement
- 18: inneres Werkzeugführungselement
- 18a: Gewebeeindringsperre
- 19: äußeres Werkzeugführungselement
- 20: Steg zwischen 17 und 18
- 21: U-förmiger Betätigungsstößel
- 22: Betätigungsschenkel von 21
- 23: schräge Betätigungsflächen von 22
- 24: Führungsschlitze in 30
- 25: Führungsbolzen von 26
- 26: Betätigungsarme
- 27: schräge Betätigungsflächen von 26
- 28: Nasen von 26
- 29: Nasen von 22
- 30: Basisplatte
- 31: Gehäuse, zweiteilig
- 32: Betätigungsstößel
- 33: Führungsstift von 34
- 34: Betätigungskörper von 32
- 35: Betätigungsarme
- 36: Betätigungsfläche von 35
- 37: Werkzeugträger
- 38: Betätigungsfläche von 34
- 39: Öffnung in 13, 31
- 40: Werkzeugführungselement, Gewebeeindringsperre
- 41: Haltesteg (41b)
- 42: Gehäuse
- 43: Führungsstifte von 46
- 44: Betätigungskörper von 45
- 45: Betätigungsstößel
- 46: Betätigungsarme
- 47: Werkzeugträger
- 48: gerade Führungsschlitze in 44
- 49: Führungsstifte am Gehäuse 42
- 50: schräge Führungsschlitze in 44
- 51: zylindrisches Gehäuse
- 51a: erstes Gehäuseteil
- 51b: zweites Gehäuseteil
- 52: Betätigungsstößel
- 53: kreisförmigerWerkzeugträger
- 54: inneres Werkzeugführungselement
- 55: runde Öffnung
- 56: äußeres Werkzeugführungselement
- 57: Druckfeder
- 60: Spendeeinrichtung
- 61: Vorratsbehälter
- 62: Spendebauteil
- 63: Öffnungen im Spendebauteil 62
- 64: Fluidleitung
- 70: Aufnahme
- β: spitzer Winkel zwischen 1,2 und 3a
- α: Winkel zwischen 1 und 2
- A: Gewebeoberfläche

## Patentansprüche

1. Applikationsmodul für eine Vorrichtung zum wiederholten Aufstechen eines organischen Gewebes, mit
- einer Penetriervorrichtung, die einen Betätigungsmechanismus und eine Anordnung von Applikationswerkzeugen (1, 2) aufweist, welche jeweils an den Betätigungsmechanismus koppeln und ein distales Werkzeugende (4) aufweisen, welches für wenigstens ein Applikationswerkzeug (1) mit einer Penetriereinrichtung gebildet ist, wobei
- die Applikationswerkzeuge (1, 2) mittels des Betätigungsmechanismus relativ zueinander verlagerbar sind zwischen einer Ausgangsstellung und einer Penetrierstellung, indem das wenigstens eine Applikationswerkzeug (1) und ein anderes Applikationswerkzeug (2) der Anordnung relativ zueinander verlagert werden, und
- beim relativen Verlagern die Wirkrichtung einer von dem wenigstens einen Applikationswerkzeug (1) bereitgestellten Applikationskraft gegenläufig ausgebildet ist zur Wirkrichtung einer von dem anderen Applikationswerkzeug (2) bereitgestellten Applikationskraft, derart, dass bei einer Kraftkomponentenzerlegung zwei parallele, aber entgegengesetzt gerichtete Kraftkomponenten gegeben sind, nämlich eine Kraftkomponente der Applikationskraft des wenigstens einen Applikationswerkzeuges (1) und eine entgegengesetzte Kraftkomponente der Applikationskraft des anderen Applikationswerkzeuges (2), und
- einer Kopplungseinrichtung, die konfiguriert ist, an eine Antriebseinrichtung zu koppeln,
**dadurch gekennzeichnet, dass** sich das wenigstens eine und das andere Applikationswerkzeug (1, 2) in Blickrichtung quer zur Wirkrichtung der von dem wenigstens einen Applikationswerkzeug (1) sowie der von dem anderen Applikationswerkzeug (2) bereitgestellten Applikationskraft zumindest in der Penetrierstellung kreuzen, nämlich im Bereich der distalen Werkzeugenden (4) selbst oder im Bereich von Verlängerungslinien der distalen Werkzeugenden (4).

2. Applikationsmodul nach Anspruch 1, **dadurch gekennzeichnet, dass** das wenigstens eine und das andere Applikationswerkzeug (1, 2) bei der Verlagerung zu der Penetrierstellung hin seitlich versetzt zueinander geführt sind.

3. Applikationsmodul nach Anspruch 1 oder 2, **dadurch gekennzeichnet, dass** mittels des Betätigungsmechanismus die Verlagerung zwischen der Ausgangsstellung und der Penetrierstellung repetierend ausführbar ist.

4. Applikationsmodul nach mindestens einem der vorangehenden Ansprüche, **dadurch gekennzeichnet, dass** eine Bewegungsbahn bei der relativen Verlagerung eine Kreisbahnbewegung und / oder eine geradlinige Bewegung umfasst.

5. Applikationsmodul nach mindestens einem der vorangehenden Ansprüche, **dadurch gekennzeichnet, dass** die Penetriereinrichtung des wenigstens einen Applikationswerkzeuges (1) zumindest in einem letzten Abschnitt der Verlagerungsbewegung zur Penetrierstellung hin entlang einer Bewegungsrichtung geführt wird, die einen vom rechten Winkel verschiedenen Winkel mit einer den distalen Werkzeugenden (4) der Applikationswerkzeuge (1, 2) im Betrieb zugeordneten Auflagefläche einnimmt.

6. Applikationsmodul nach mindestens einem der vorangehenden Ansprüche, **dadurch gekennzeichnet, dass** das wenigstens eine Applikationswerkzeug (1) und / oder das andere Applikationswerkzeug (2) bei der Verlagerungsbewegung zwischen der Ausgangsstellung und der Penetrierstellung wenigstens abschnittsweise in einer zugeordneten Werkzeugführung (70) geführt werden.

7. Applikationsmodul nach mindestens einem der vorangehenden Ansprüche, **dadurch gekennzeichnet, dass** das wenigstens eine Applikationswerkzeug (1) und / oder das andere Applikationsgegenwerkzeug (2) mit einem Kammbauteil (9) gebildet sind, an dem mehrere nebeneinander liegende Applikationselemente angeordnet sind.

8. Applikationsmodul nach mindestens einem der vorangehenden Ansprüche, **dadurch gekennzeichnet, dass** ein Hub der Verlagerungsbewegung des wenigstens einen Applikationswerkzeuges (1) und / oder des anderen Applikationswerkzeuges (2) einstellbar ist.

9. Applikationsmodul nach mindestens einem der vorangehenden Ansprüche, **dadurch gekennzeichnet, dass** im Bereich der Anordnung von Applikationswerkzeugen (1, 2) und / oder benachbart hierzu eine Gewebeanschlag- oder Gewebeführungsfläche gebildet ist.

10. Applikationsmodul nach mindestens einem der vorangehenden Ansprüche, **dadurch gekennzeichnet, dass** die Applikationswerkzeuge (1, 2) an einem gemeinsamen Basisbauteil gebildet sind.

11. Applikationsmodul nach mindestens einem der vorangehenden Ansprüche, **dadurch gekennzeichnet, dass** das distale Werkzeugende (4) der Applikationswerkzeuge (1, 2) zumindest in der Ausgangsstellung ganz oder teilweise in ein Gerätegehäuse eingefahren ist.

12. Applikationsmodul nach mindestens einem der vorangehenden Ansprüche, **dadurch gekennzeichnet, dass** eine Spendeeinrichtung (60) vorgesehen ist, die an der Penetriervorrichtung angeordnet und geeignet ist, einen zu applizierenden Wirkstoff auszubringen.

13. Vorrichtung zum wiederholten Aufstechen eines organischen Gewebes, mit einem Applikationsmodul nach mindestens einem der vorangehenden Ansprüche und einer maschinellen Antriebseinrichtung, die an die Kopplungseinrichtung und den Betätigungsmechanismus koppelt und geeignet ist, das relative Verlagern des wenigstens einen Applikationswerkzeuges (1) zwischen der Ausgangsstellung und der Penetrierstellung wiederholt mit einer Betriebsfrequenz zu bewirken.

## Claims

1. Application module for a device for the repeated piercing of an organic tissue with:
- a penetration device which has an operating mechanism and an arrangement of application tools (1, 2) each of which is connected to the operating mechanism and has a distal tool end (4) which is formed for at least one application tool (1) with a penetration device, whereby
- by way of the operating mechanism the application tools (1, 2) can be displaced relative to each other between an initial position and a penetration position in that the at least one application tool (1) and another application tool (2) of the arrangement are moved relative to each other, and
- during the relative movement the effect direction of an application force provided by the at least one application tool (1) is counter to the effect direction of the application force provided by the other application tool (2) such that when resolving into force components two force components result that are parallel but work in counter directions, these being one force component of the application force of the at least one application tool (1) and a counter force component of the application force of the other application tool (2), and
- a coupling device that is configured to connect to a mechanical actuation device, **characterized in that** viewed in the direction perpendicular to the application force provided by the at least one application tool (1) as well as to that provided by the other application tool (2) the at least one and the other application tool (1, 2) cross each other at least in the penetration position, namely in the area of the distal tool ends (4) themselves or in the area of projection lines of the distal tool ends (4).

2. Application module according to claim 1, wherein during the movement towards the penetration position the at least one and the other application tool (1, 2) are guided laterally offset with regard to each other.

3. Application module according to claim 1 or 2, wherein by means of the operating mechanism the movement between the initial position and the penetration position can be repeatedly carried out.

4. Application module according to at least one of the preceding claims, wherein a movement path during the relative displacement comprises a curved movement and/or a straight movement.

5. Application module according to at least one of the preceding claims, wherein the penetration device of the at least one application tool (1) is guided at least in a last section of the displacement movement to the penetration position along a direction of movement which assumes an angle, which differs from a right angle with the contact surface assigned to the distal tool ends (4) of the application tools (1, 2) during operation.

6. Application module according to at least one of the preceding claims, wherein during the displacement movement between the initial position and the penetration position the at least one application tool (1) and/or the other application tool (2) are guided at least in sections in an assigned tool guide (70).

7. Application module according to at least one of the preceding claims, wherein the at least one application tools (1) and/or the other application counter tool (2) are formed with a comb component (9) on which several adjacent application elements are arranged.

8. Application module according to at least one of the preceding claims, wherein a stroke length of the displacement movement of the at least one application tool (1) and/or the other application tools (2) can be adjusted.

9. Application module according to at least one of the preceding claims, wherein in the area of the arrangement of the application tools (1, 2) and/or adjacent thereto a tissue contact or tissue guide surface is formed.

10. Application module according to at least one of the preceding claims, wherein the application tools (1, 2) are formed on a common basic component.

11. Application module according to at least one of the preceding claims, wherein the distal tool end (4) of the application tools (1, 2) is partially or fully retracted into a housing at least in the initial position.

12. Application module according to at least one of the preceding claims, wherein a dispensing device (60) is provided which is arranged on the penetration device and is suitable for dispensing a substance to be applied.

13. Device for the repeated piercing of an organic tissue comprising an application module according to at least one of the preceding claims and a mechanical actuation device that connects to the coupling device and is suitable for bringing about the relative displacement between the initial position and the penetration position of the at least one application tool (1) repeatedly with an operating frequency.

## Revendications

1. Module d'application pour un dispositif destiné au perçage répété d'un tissu organique, avec
- un dispositif de pénétration qui comporte un mécanisme de manoeuvre et un agencement d'outils d'application (1, 2), lesquels sont accouplés chacun au mécanisme de manoeuvre et comportent une extrémité distale (4) d'outil, laquelle est formée pour au moins un outil d'application (1) avec un système de pénétration,
- à l'aide du mécanisme de manoeuvre, les outils d'application (1, 2) étant déplaçables les uns par rapport aux autres entre une position de départ et une position de pénétration en ce qu'on déplace l'un par rapport à l'autre l'au moins un outil d'application (1) et un autre outil d'application (2) de l'agencement et
- lors du déplacement relatif, la direction d'action d'une force d'application mise à disposition par l'au moins un outil d'application (1) étant conçue à contresens de la direction d'action d'une force d'application mise à disposition par l'autre outil d'application (2), de telle sorte que lors d'une décomposition d'une composante de force, deux composantes de force parallèle, mais en direction opposée soient données, à savoir une composante de force de la force d'application de l'au moins un outil d'application (1) et une composante de force opposée de la force d'application de l'autre outil d'application (2) et
- un système de couplage qui est configuré pour s'accoupler sur un système d'entraînement,
**caractérisé en ce que** l'au moins un et l'autre outils d'application (1, 2) se croisent au moins dans la position de pénétration dans la direction de visée à la transversale de la direction d'action de la force d'application mise à disposition par l'au moins un outil d'application (1), ainsi que de la force d'application mise à disposition par l'autre outil d'application (2), à savoir dans la zone des extrémités distales (4) d'outils même ou dans la zone des lignes de prolongement des extrémités distales (4) d'outils.

2. Module d'application selon la revendication 1, **caractérisé en ce que** lors du déplacement dans la position de pénétration, l'au moins un et l'autre outils d'application (1, 2) sont guidés en étant latéralement déportés l'un par rapport à l'autre.

3. Module d'application selon la revendication 1 ou la revendication 2, **caractérisé en ce qu'**à l'aide du mécanisme de manoeuvre, le déplacement entre la position de départ et la position de pénétration est réalisable de manière répétitive.

4. Module d'application selon au moins l'une quelconque des revendications précédentes, **caractérisé en ce qu'**une trajectoire de déplacement lors du déplacement relatif comprend un déplacement en trajectoire circulaire et/ou un déplacement rectiligne.

5. Module d'application selon au moins l'une quelconque des revendications précédentes, **caractérisé en ce qu'**au moins dans une dernière partie du mouvement de déplacement vers la position de pénétration, le système de pénétration de l'au moins un outil d'application (1) est guidé le long d'une direction de déplacement qui adopte un angle différent de l'angle droit avec une surface d'appui associée en service aux extrémités distales (4) des outils d'application (1, 2).

6. Module d'application selon au moins l'une quelconque des revendications précédentes, **caractérisé en ce que** lors du déplacement entre la position de départ et la position de pénétration, l'au moins un outil d'application (1) et/ou l'autre outil d'application (2) sont guidés au moins en partie dans un guide d'outil (70) associé.

7. Module d'application selon au moins l'une quelconque des revendications précédentes, **caractérisé en ce que** l'au moins un outil d'application (1) et/ou l'autre outil d'application (2) sont formés avec un élément constitutif en peigne (9) sur lequel sont placés plusieurs éléments d'application placés côte à côte.

8. Module d'application selon au moins l'une quelconque des revendications précédentes, **caractérisé en ce qu'**une course du mouvement de déplacement de l'au moins un outil d'application (1) et/ou de l'autre outil d'application (2) sont réglables.

9. Module d'application selon au moins l'une quelconque des revendications précédentes, **caractérisé en ce que** dans la région de l'agencement d'outils d'application (1, 2) ou dans son voisinage est formée un surface de butée du tissu ou une surface de guidage du tissu.

10. Module d'application selon au moins l'une quelconque des revendications précédentes, **caractérisé en ce que** les outils d'application (1, 2) sont formés sur un élément constitutif commun.

11. Module d'application selon au moins l'une quelconque des revendications précédentes, **caractérisé en ce que** l'extrémité distale (4) d'outil des outils d'application (1, 2) est totalement ou partiellement rentrée dans un boîtier d'appareil, au moins dans la position de départ.

12. Module d'application selon au moins l'une quelconque des revendications précédentes, **caractérisé en ce qu'**il est prévu un système distributeur (60) qui est placé sur le dispositif de pénétration et qui est apte à délivrer un principe actif à appliquer.

13. Dispositif destiné au perçage répété d'un tissu organique, avec un module selon au moins l'une quelconque des revendications précédentes et un système d'entraînement mécanique qui est accouplé au système de couplage et au mécanisme de manoeuvre et qui est adapté pour provoquer de manière répétée le déplacement relatif de l'au moins un outil d'application (1) entre la position de départ et la position de pénétration, avec une fréquence de service.
